(19) Europäisches Patentamt
European Patent Office
Office européen des brevets

(11) **EP 3 089 752 B1**

(12) **FASCICULE DE BREVET EUROPEEN**

(45) Date de publication et mention
de la délivrance du brevet:
**30.10.2019 Bulletin 2019/44**

(21) Numéro de dépôt: **14796180.9**

(22) Date de dépôt: **23.09.2014**

(51) Int Cl.:
*A61K 8/39* *(2006.01)*     *A61Q 1/10* *(2006.01)*
*A61K 8/86* *(2006.01)*     *A61K 8/02* *(2006.01)*
*A61K 8/37* *(2006.01)*     *A61K 8/49* *(2006.01)*
*A61K 8/81* *(2006.01)*

(86) Numéro de dépôt international:
**PCT/FR2014/052367**

(87) Numéro de publication internationale:
**WO 2015/052398 (16.04.2015 Gazette 2015/15)**

(54) **COMPOSITION COSMÉTIQUE DE REVÊTEMENT DES FIBRES KÉRATINIQUES**

KOSMETISCHE ZUSAMMENSETZUNG ZUR BESCHICHTUNG VON KERATINFASERN

COSMETIC COMPOSITION FOR COATING KERATIN FIBRES

(84) Etats contractants désignés:
**DE ES FR GB IT**

(30) Priorité: **11.10.2013 FR 1359889**

(43) Date de publication de la demande:
**09.11.2016 Bulletin 2016/45**

(73) Titulaire: **L'Oréal**
**75008 Paris (FR)**

(72) Inventeurs:
• **ILEKTI, Philippe**
**F-94700 Maisons-Alfort (FR)**
• **JAGER LEZER, Nathalie**
**F-91370 Verrieres-le-buisson (FR)**

(74) Mandataire: **L'Oreal**
**Service D.I.P.I.**
**9, rue Pierre Dreyfus**
**92110 Clichy (FR)**

(56) Documents cités:
**EP-A1- 1 920 759     EP-A2- 1 396 259**
**FR-A1- 2 960 151**

**Description**

La présente invention concerne une composition cosmétique de revêtement des fibres kératiniques, et en particulier des cils ou sourcils. En particulier, ladite composition de cosmétique est une composition de maquillage et éventuellement de soin des cils. La présente invention concerne également un procédé de revêtement des fibres kératiniques, en particulier un procédé de maquillage et éventuellement de soin des cils. La présente invention concerne également des utilisations particulières.

La composition mise en oeuvre peut notamment se présenter sous la forme d'un produit pour les cils tel qu'un mascara, ou d'un produit pour les sourcils. Plus préférentiellement, l'invention porte sur un mascara. Par « mascara », on entend une composition destinée à être appliquée sur les cils : il peut s'agir d'une composition de maquillage des cils, une base de maquillage des cils (aussi appelée base coat), une composition à appliquer sur un mascara, dite encore top-coat, ou bien encore une composition de traitement cosmétique des cils. Le mascara est plus particulièrement destiné aux cils d'êtres humains, mais également aux faux-cils.

Les mascaras comprennent conventionnellement une quantité non négligeable en cires et sont communément préparés selon deux types de formulation : les mascaras aqueux dits mascaras crèmes, sous forme de dispersion de cires dans l'eau ; les mascaras anhydres ou à faible teneur en eau, dits mascaras water-proofs, sous forme de dispersions de cires dans des solvants organiques.

Généralement, les mascaras anhydres ont une bonne tenue à l'eau mais le niveau de volume est généralement faible et le démaquillage difficile, tandis que les mascaras aqueux ont une tenue à l'eau plus faible mais un niveau de volume important et un démaquillage plus facile.

La présente demande concerne plus spécifiquement les mascaras dits aqueux.

L'application de mascara vise en particulier à augmenter le volume des cils et en conséquence augmenter l'intensité du regard. Pour cela, il existe de nombreux mascaras épaississants ou volumateurs dont le principe consiste à déposer le maximum de matière sur les cils de manière à obtenir cet effet volumateur (ou chargeant). C'est en particulier à travers la quantité de particules (notamment les cires), que peuvent être ajustées les spécificités d'application recherchées pour les compositions, comme par exemple leur fluidité ou consistance, ainsi que leur pouvoir épaississant (encore appelé pouvoir chargeant ou maquillant).

Toutefois, en premier lieu il s'avère que l'évolution de la texture d'une composition conventionnelle de mascara comprenant des cires peut être parfois difficilement contrôlable et rédhibitoire en termes de produit, devenant parfois solide deux mois à 45°C et ainsi n'être plus applicable.

Ce problème est notamment rencontré avec les voies formulatoires conventionnelles des mascaras aqueux ne permettent pas de dépasser une teneur en extrait sec élevé, par exemple supérieure ou égale à 42 %, sous peine de texture trop épaisse.

La demande EP 1 920 759 propose de structurer les compositions de mascaras à l'aide de cires, afin d'obtenir une texture épaisse et un dépôt volumateur. Afin de disperser de façon homogène ces cires, un système de tensioactifs particuliers doit être utilisé, comprenant un phosphate d'alkyle et un éther d'alcool gras et de polyéthylène glycol présentant une HLB strictement inférieure à 8. Cette demande illustre plusieurs exemples de compositions de mascara comprenant 20.14 % en poids de cires, pour au maximum 10 % en poids total de tensioactifs, par rapport au poids total de la composition, avec les tensioactifs jouant essentiellement un rôle d'émulsification de ces cires.

La demande FR 2 960 151 se propose quant à elle de favoriser la tenue et la résistance à l'effritement par l'emploi d'un système de tensioactifs particulier comprenant un phosphate d'alkyle et un éther d'alcool gras et de polyéthylène glycol présentant une HLB strictement inférieure à 8, associé à une phase cireuse particulière. Cette demande illustre plusieurs exemples de des compositions de mascara comprenant plus de 25.59 % en poids de cires, émulsionnées grâce au système tensioactif précité.

Un but de la présente invention est ainsi d'obtenir une nouvelle voie formulatoire d'un mascara présentant une stabilité améliorée dans le temps et/ou à la température, par exemple stable pendant 2 mois à 45°C.

Par ailleurs, il est connu d'utiliser des teneurs pondérales élevées de particules de polymère dans le but d'apporter une résistance à l'eau du dépôt sur le cil. La majorité des mascaras smudgeproof sont ainsi préparés. Cependant de telles compositions sont peu agréables à l'application car le playtime est court : le nombre de coup de brosse reste limité, l'application devient difficile après plusieurs coups de brosses. L'association de phases lamellaires Lβ avec des particules de polymères filmogènes nous permet de réaliser des textures agréables à l'application.

Un but de la présente invention est ainsi de proposer un mascara permettant d'augmenter le délai de « playtime », en particulier de pouvoir superposer les couches avec un nombre de coup de brosse supérieur ou égale à 20, mieux supérieure ou égale à 30.

Un but de la présente demande est plus particulièrement de proposer un mascara de préférence à fort extrait sec, par exemple supérieur ou égale à 42%.

Plus particulièrement, un but de la présente invention consiste à stabiliser un mascara sans déphasage dans le temps et /ou aux UV et/ou à la lumière.

Un but de la présente demande est plus particulièrement de proposer un mascara stable, présentant une texture suffisamment épaisse pour obtenir un dépôt chargeant, de consistance satisfaisante, permettant une application facile sur les cils et un dépôt régulier, c'est à dire lisse et homogène, de préférence même après deux mois stockés à 4°C.

Un but de la présente demande est également de proposer un mascara stable, présentant une texture suffisamment épaisse pour obtenir un dépôt chargeant, de consistance satisfaisante, permettant une application facile sur les cils et un dépôt régulier, c'est à dire lisse et homogène, de préférence même après deux mois stockés à 45°C.

Un but de la présente demande est plus particulièrement de proposer un mascara stable, présentant une texture suffisamment épaisse pour obtenir un dépôt chargeant, de consistance satisfaisante, permettant une application facile sur les cils et un dépôt régulier, c'est à dire lisse et homogène, de préférence même après deux mois stockés à des températures oscillant entre 4°C et 45°C.

Un but de la présente demande est plus particulièrement de proposer un mascara dans lequel les pigments sont dispersés de façon homogène.

Un but de la présente demande est plus particulièrement de proposer un mascara agréable à l'application.

Un but de la présente invention est plus particulièrement de proposer une composition de revêtement des fibres kératiniques permettant une bonne séparation des cils lors de son application, sans formation de faisceaux de cils, et ce en assurant un dépôt de matière lisse et régulier (sans amas de composition).

Un but de la présente invention est plus particulièrement d'obtenir une composition de revêtement des fibres kératiniques, de préférence un mascara, possédant de bonnes propriétés d'application en terme de glissant et de playtime (redépôt, retouche).

Un but de la présente invention est encore d'obtenir une composition de revêtement des fibres kératiniques, de préférence un mascara, donnant lieu à un effet volume sur les cils, de préférence ce malgré la présence d'une faible quantité de cires, voire ce malgré l'absence de cires.

Un but de la présente invention est encore d'obtenir une composition de revêtement des fibres kératiniques, de préférence un mascara, possédant une bonne tenue sur les cils.

Un but de la présente invention est encore d'obtenir une composition de revêtement des fibres kératiniques, de préférence un mascara, possédant de bonnes propriétés d'allongement pour les cils revêtues d'une telle composition.

Un but de la présente invention est d'encore obtenir une composition de revêtement des fibres kératiniques, de préférence un mascara, possédant de bonnes propriétés de recourbement pour les cils revêtues d'une telle composition.

Un but de la présente invention est encore d'obtenir une composition de revêtement des fibres kératiniques, de préférence un mascara, possédant une bonne adhérence sur les cils.

En particulier, un but de la présente invention est de réaliser une composition de revêtement des fibres kératiniques de bonne tenue, résistant bien aux frottements et / ou à l'eau, notamment à l'humidité ambiante, aux larmes, à la sueur et/ou au sébum, tout en permettant un démaquillage facile.

En conséquence, la présente invention a pour objet une composition cosmétique de revêtement des fibres kératiniques selon la revendication 1.

De manière surprenante et inattendue, les inventeurs de la présente demande ont résolu ce(s) problème(s) au moyen d'une telle composition. En particulier, une composition conforme à l'invention donne lieu à une composition, pouvant être riche en extrait sec, notamment en particules de polymère(s) filmogène(s), stable, ayant une dispersion de particules homogène et régulière, même au bout de 2 mois que ce soit à 45°C ou à 4°C. Une telle composition est encore agréable à l'application, confortable et présente un effet volume. Outre une tenue à l'eau renforcée, cette composition présente un playtime amélioré malgré la présence de particules de polymère(s) filmogène(s).

Selon les inventeurs, une phase lamellaire Lβ en association avec une dispersion de particules de polymère(s) filmogène(s) telle que décrite ci-dessus permet un excellent compromis texture et cosméticité, conférant un effet chargeant, résistant (à l'eau en particulier), et stable dans le temps, même au bout de 2 mois que ce soit à 45°C ou à 4°C, éventuellement tout en se dispensant de cire(s) pourtant conventionnellement utilisée(s) en mascara pour obtenir un bon effet chargeant. Cette nouvelle voie formulatoire permet ainsi de façon surprenante de se départir de l'utilisation de cires, pourtant conventionnelles dans un mascara. De plus, cette phase lamellaire Lβ permet de façon inattendue un « playtime » amélioré, même en présence d'une dispersion de particules de polymère(s) filmogène(s).

Un système conforme à l'invention peut éventuellement être essentiellement constitué de, voire peut être constitué de :

 i) au moins un tensioactif non ionique de valeur HLB à 25°C inférieure à 8, et
 ii) au moins un tensioactif non ionique de valeur HLB à 25°C supérieure ou égale à 8, formant ensemble une phase lamellaire Lβ.

La présente invention a également pour objet un procédé de revêtement des fibres kératiniques, en particulier de maquillage des cils, comprenant une étape d'application d'une composition cosmétique de revêtement telle que précédemment définie.

Selon des modes de réalisation particuliers préférés de la présente invention concernant à la fois les compositions et les procédés sus-décrits et visant à résoudre au moins l'un des problèmes susmentionnés :

- la phase aqueuse représente de 30 à 70% en poids, de préférence de 40 à 60 % en poids, par rapport au poids total de la composition ;
- la composition présente une phase aqueuse continue ;
- le (les) tensioactif(s) non ionique(s) de valeur HLB à 25°C supérieure ou égale à 8, ont de préférence une valeur HLB à 25°C supérieure ou égale à 10 ;
- le (les) au moins un parmi le (les) tensioactif(s) non ionique(s) de valeur HLB à 25°C inférieure à 8 est (sont) choisi(s) parmi :

  * les esters et éthers d'oses éventuellement (poly)oxyalkylénés, de préférence (poly)oxyalkyléné;
  * les esters d'acides gras, notamment en C8-C24, et de préférence en C16-C22, et de polyol éventuellement (poly)oxyalkyléné, de préférence (poly)oxyalkyléné, notamment de glycérol (poly)oxyalkyléné ou de sorbitol oxyalkyléné, de préférence de glycérol (poly)oxyalkyléné ;
  * les alcools éventuellement (poly)oxyalkylénés, de préférence (poly)oxyalkylénés ;
  * et leurs mélanges ; de préférence parmi les alcools éventuellement (poly)oxyalkylénés, de préférence (poly)oxyalkylénés ;

- le (les) au moins un parmi le (les) tensioactif(s) non ionique(s) de valeur HLB à 25°C inférieure à 8 comprend un alcool éventuellement (poly)oxyalkyléné, de préférence (poly)oxyalkyléné comprenant un éther d'alcool gras en $C_8$-$C_{24}$ et de polyéthylène glycol, ledit éther comprenant de 1 à 10, mieux entre 2 et 6, motifs d'éthylène glycol.
- le (les) tensioactif(s) non ionique(s) de valeur HLB à 25°C supérieure ou égale à 8, de préférence supérieure ou égale à 10, est (sont) choisi(s) parmi :

  * les éthers de glycérol (poly)oxyalkylénés,
  * les alcools (poly)oxyalkylénés,
  * les esters d'acide gras et de polyéthylène glycol (poly)oxyalkylénés,
  * les esters d'acide gras et d'éthers de glycérol (poly)oxyalkylénés,
  * les esters d'acide gras et d'éthers de sorbitol (poly)oxyalkylénés,

  et leur(s) mélange(s) ; de préférence parmi les alcools (poly)oxyalkylénés ;
- le (les) au moins un parmi le (les) tensioactif(s) non ionique(s) de valeur HLB à 25°C supérieure ou égale à 8 comprend un alcool (poly)oxyalkyléné comprenant au moins un éther d'alcool gras en $C_8$-$C_{24}$ et de polyéthylène glycol, ledit éther comprenant plus de 10 motifs d'éthylène glycol, mieux entre 15 et 200 motifs d'éthylène glycol ;
- le (les) tensioactif(s) non ionique(s) de valeur HLB à 25°C inférieure à 8 et le (les) tensioactif(s) non ionique(s) de valeur HLB à 25°C supérieure ou égale à 8, répondant de préférence tous deux à la formule (I), sont présents à une teneur totale supérieure ou égale à 15 %, en particulier comprise entre 16 et 40% en poids, mieux entre 18 et 30% en poids, par rapport au poids total de la composition ;
- le (les) tensioactif(s) non ionique(s) de valeur HLB à 25°C inférieure à 8 et le (les) tensioactif(s) non ionique(s) de valeur HLB à 25°C supérieure ou égale à 8, répondant de préférence tous deux à la formule (I), sont présents à une teneur totale respective telle que le rapport pondéral du (des) tensioactif(s) non ionique(s) de valeur HLB à 25°C inférieure à 8 sur le (les) tensioactif(s) non ionique(s) de valeur HLB à 25°C supérieure ou égale à 8 va de 1/5 à 5, de préférence de 1/3 à 3, de préférence de 2/3 à 3/2 ;
- les particules de polymères filmogènes introduites sous forme de dispersion(s) aqueuse(s) dans ladite composition présentent dans ladite composition une taille moyenne exprimée en diamètre « effectif » moyen en volume D[4,3] inférieure ou égale à 5 μm, préférentiellement inférieure ou égale à 2 μm, plus préférentiellement encore à 1 μm, par exemple compris entre 0.01 et 5 μm, et plus préférentiellement entre 0.1 et 2 μm ;
- les particules de polymère(s) filmogène(s) présente(s) sous forme de dispersion(s) aqueuse(s) sont présentes selon une teneur supérieure ou égale à 5 % en poids, de préférence à 10 % en poids, plus préférentiellement comprise entre 10 et 30 % en poids, par rapport au poids total de la composition ;
- les particules de polymère(s) filmogène(s) présente(s) sous forme de dispersion(s) aqueuse(s) est (sont) choisi(s) parmi les polymères synthétiques, de type radicalaire ou de type polycondensat, les polymères d'origine naturelle, et leurs mélanges ;
- les particules de polymère(s) filmogène(s) présente(s) sous forme de dispersion(s) aqueuse(s) est (sont) choisi(s) parmi les dispersions de polymères acryliques, les dispersions de polyuréthanne, les dispersions de sulfopolyesters, les dispersions vinyliques, les dispersions de polyvinyl acétate, les dispersions de terpolymère vinyl pyrrolidone, diméthylaminopropyl méthacrylamide et chlorure de lauryldiméthylpropylméthacrylamidoammonium, les dispersions de polymère hybrides polyuréthane/polyacryliques, les dispersions de particules de type cœur-écorce et leurs mélanges, de préférence parmi les dispersions de polymères acryliques, dispersions de polymère hybrides polyuréthane/polyacryliques, et leurs dérivés, et leur(s) mélange(s), préférentiellement parmi les dispersions de polymères acryliques, en particulier styrène-acrylique, et les dispersions de polyuréthanne, en particulier polyester-polyuréthanne, et leurs dérivés, et leur(s) mélange(s) ;

- la teneur totale en particules de polymère(s) filmogène(s) et la teneur totale en système tensioactif sont telles que le rapport pondéral des particules de polymère(s) filmogène(s) / le système tensioactif est inférieur ou égal à 1.75, de préférence inférieur ou égal à 1.5, en particulier compris entre 1/2 et 1.25 ;
- la composition comprend au moins une charge sphérique, de préférence la (les) charge(s) sphérique(s) est (sont) choisie(s) parmi :

  • des poudres de silice ;
  • des poudres de (co)polymères acryliques, et leurs dérivés, en particulier des poudres de (co)polymère acrylate, et leurs dérivés ;
  • des poudres de polyuréthane ;
  • des poudres de silicone ;
  • des poudres de polyamide ;

  et leur(s) mélange(s), de préférence parmi les poudres de (co)polymères acryliques, et leurs dérivés, en particulier des poudres de (co)polymère acrylate, les poudres de polyuréthane, et leur(s) mélange(s) ;
- la (les) charge(s) sphérique(s) est (sont) présente(s) à une teneur totale supérieure ou égale à 5 % en poids, par rapport au poids total de la composition, par exemple compris entre 8 et 30 % en poids, de préférence comprise entre 8 et 25 % en poids, mieux entre 10 et 20 % en poids, par rapport au poids total de la composition ;
- la composition comprend au moins une charge lamellaire, de préférence la (les) charge(s) lamellaire(s) est (sont) choisie(s) parmi le talc, le mica naturel ou synthétique, certaines silices, les argiles tels que les silicate de magnésium et d'aluminium, le kaolin, la bentone, le carbonate de calcium et l'hydrogéno-carbonate de magnésium, l'hydroxyapatite, le nitrure de bore, la fluorphlogopite, une poudre N-Lauroyl Lysine, des poudres de perlite et leur(s) mélange(s), plus préférentiellement encore la (les) charge(s) lamellaire(s) est (sont) choisie(s) parmi le talc, le mica, la fluorphlogopite, les argiles tels que le silicate d'aluminium et de magnésium, une poudre N-Lauroyl Lysine, et leur(s) mélange(s) ;
- la (les) charge(s) lamellaire(s) est (sont) présente(s) à une teneur totale supérieure ou égale à 5 % en poids, par rapport au poids total de la composition, par exemple compris entre 8 et 30 % en poids, de préférence comprise entre 8 et 25 % en poids, mieux entre 10 et 20 % en poids, par rapport au poids total de la composition ;
- ladite composition est exempte d'huile ou solvant organique ;
- ladite composition comprend une teneur en extrait sec supérieure ou égale à 42 %, préférentiellement à 45 %, plus préférentiellement à 48 %, voire à 50 % ;
- ladite composition comprend une teneur en extrait sec inférieure à 60 % ;
- ladite composition comprend au moins un polymère filmogène hydrosoluble, plus préférentiellement ladite composition est exempt de polymère filmogène hydrosoluble ;
- ladite composition comporte au moins une matière colorante choisie parmi une ou plusieurs matière(s) pulvérulente(s), de préférence des oxydes métalliques, et en particulier des oxydes de fer ;
- Le (les) oxyde(s) métallique(s) est (sont) de préférence présent(s) à une teneur supérieure ou égale à 5% en poids par rapport au poids total de la composition, avantageusement comprise inclusivement entre 6 et 22% en poids par rapport au poids total de la composition ;
- ladite composition comprend au moins un gélifiant hydrophile et/ou lipophile, de préférence au moins un gélifiant hydrophile ;
- ladite composition présente une viscosité à 25°C allant de 5 à 50 Pa.s, en particulier mesurée à l'aide d'un appareil Rhéomat RM100® ;
- ladite composition peut être une composition de maquillage, une base de maquillage ou « base coat », une composition dite « top-coat » à appliquer sur un maquillage ;

D'autres caractéristiques, propriétés et avantages de la présente invention apparaîtront plus clairement à la lecture de la description et des exemples qui suivent.

## Phase aqueuse

La composition selon l'invention comprend une phase aqueuse, qui forme avantageusement une phase continue de la composition.

Par composition à phase continue aqueuse, on entend que la composition présente une conductivité, mesurée à 25 °C, supérieure ou égale à 23 µS/cm (microSiemens/cm), la conductivité étant mesurée par exemple à l'aide d'un conductimètre MPC227 de Mettler Toledo et d'une cellule de mesure de conductivité Inlab730. La cellule de mesure est immergée dans la composition, de façon à éliminer les bulles d'air susceptibles de se former entre les 2 électrodes de la cellule. La lecture de la conductivité est faite dès que la valeur du conductimètre est stabilisée. Une moyenne est réalisée sur au moins 3 mesures successives.

La phase aqueuse comprend de l'eau. Elle peut également comprendre au moins un solvant hydrosoluble.

Par "solvant hydrosoluble", on désigne dans la présente invention un composé liquide à température ambiante et miscible à l'eau.

Les solvants hydrosolubles utilisables dans les compositions selon l'invention peuvent en outre être volatils.

Parmi les solvants hydrosolubles pouvant être utilisés dans les compositions conformes à l'invention, on peut citer notamment les monoalcools inférieurs ayant de 1 à 5 atomes de carbone tels que l'éthanol et l'isopropanol, les glycols ayant de 2 à 8 atomes de carbone tels que l'éthylène glycol, le propylène glycol, le 1,3-butylène glycol et le dipropylène glycol.

La phase aqueuse (eau et éventuellement le solvant miscible à l'eau) est généralement présente dans la composition selon la présente demande en une teneur allant de 30 à 70 % en poids, par rapport au poids total de la composition, de préférence allant de 40 à 60 % en poids par rapport au poids total de la composition. Cette teneur en phase aqueuse inclut non seulement l'eau provenant des dispersions aqueuses de polymères filmogènes, et le cas échéant l'eau délibérément ajoutée à la composition.

### Extrait sec

La composition selon l'invention comprend avantageusement une teneur en extrait sec supérieure ou égale à 42%, en particulier à 45 %, voire à 48 % et préférentiellement à 50%, et avantageusement inférieure ou égale à 60%.

Au sens de la présente invention, la « *teneur en extrait sec* », désigne la teneur en matière non volatile.

La quantité d'extrait sec (abrégé ES) d'une composition selon l'invention est mesurée au moyen d'un dessiccateur à halogène commercial « HALOGEN MOISTURE ANALYZER HR 73 » de chez METTLER TOLEDO. La mesure se fait sur la base de la perte de poids d'un échantillon séché par chauffage halogène et représente donc le pourcentage de matière résiduelle une fois que l'eau et les matières volatiles se sont évaporées.

Cette technique est parfaitement décrite dans la documentation de l'appareil fournie par METTLER TOLEDO.

Le protocole de mesure est le suivant :

On étale environ 2 g de la composition, ci-après l'échantillon, sur une coupelle métallique que l'on introduit dans le dessiccateur à halogène mentionné ci-dessus. L'échantillon est alors soumis à une température de 120 °C jusqu'à obtenir un poids constant. La Masse Humide de l'échantillon, correspondant à sa masse initiale, et la Masse Sèche de l'échantillon, correspondant à sa masse après chauffage halogène, sont mesurées au moyen d'une balance de précision.

L'erreur expérimentale liée à la mesure est de l'ordre de plus ou moins 2 %. La teneur en Extrait Sec est calculée de la manière suivante :

Teneur en Extrait Sec (exprimé en % en poids) = 100 x (Masse Sèche / Masse Humide).

Une composition selon l'invention comprend des particules de cires, de polymère(s) filmogène(s), et au moins un système tensioactif particulier.

### Phase lamellaire Lβ

Ainsi, la présente demande a pour objet une composition contenant dans un milieu aqueux, un système tensioactif, organisé sous forme d'une phase lamellaire Lβ, ou phase paracristalline Lβ, ou phase lamellaire gel.

Cette composition est stable à température ambiante de 25°C. ayant une viscosité allant préférentiellement de 5 à 50 Pa.s, mesurée à la température ambiante de 25°C à l'aide d'un Rhéomat RM100®.

On entend par phase lamellaire gel ou phase paracristalline Lβ, une phase dans laquelle les molécules de tensioactifs et/ou plus généralement de composés amphiphiles s'organisent sous forme de couches bimoléculaires espacées par des feuillets aqueux. Au sein des couches bimoléculaires, les molécules sont réparties selon une géométrie hexagonale, leurs chaînes hydrocarbonées sont dans un état cristallin et sont orientées perpendiculairement au plan des couches bimoléculaires mais n'ont pas d'orientation spécifique les unes par rapport aux autres dans le plan de ces couches.

Les phases paracristallines Lβ sont des phases métastables au sein desquelles les chaînes grasses sont à l'état solide et sont disposées de manière aléatoire les unes par rapport aux autres, contrairement aux phases paracristallines micellaire, hexagonale, cubique et lamellaire fluide (La) au sein desquelles les chaînes grasses sont à l'état liquide, et contrairement aux phases cristallines au sein desquelles les chaînes grasses sont à l'état solides et orientées de manière ordonnée les unes par rapport aux autres. La demanderesse a trouvé un système tensioactif particulier permettant d'obtenir une phase paracristalline Lβ stable, et ainsi des compositions cosmétique de revêtement des fibres kératiniques, en particulier des cils, stables et agréable à l'application en utilisant un système particulier de type de tensioactifs selon des teneurs particulières.

Pour identifier la phase lamellaire gel ou phase paracristalline Lβ du système émulsionnant présent dans la composition de l'invention, on peut utiliser différentes techniques, et notamment la technique de diffraction des rayons-X aux grands angles.

*Diffraction des rayons X aux grands angles (Wide angle X-ray Scattering - WAXS)*

Des diagrammes de rayons X ont été enregistrés par un détecteur à plaques d'images Mar345 (Maresearch, Norderstedt, Allemagne), monté sur un générateur de rayons X à anode rotative FR591 (Bruker, Courtaboeuf, France), utilisé à 50 kV et à 50 mA. Le rayonnement CuKα monochromatique ($\lambda$ = 1,541 Å) était focalisé avec une tache focale de 350 μm à 320 mm par une réflexion double sur un miroir Montel multicouche de section transversale elliptique (Incoatec, Geesthacht, Allemagne). Le faisceau était défini sous vide par quatre fentes en carbone-tungstène motorisées (JJ-Xray, Roskilde, Danemark) positionnées devant le miroir (500 μm). Quatre fentes de protection supplémentaires étaient placées au point focal à une distance de séparation des fentes de 220 mm. Le flux en aval des fenêtres de sortie en mica était de $3 \times 10^8$ photons/s. Un arrêt de faisceau en fil métallique circulaire de 2 mm de diamètre était placé dans l'air à 150 mm en aval de l'échantillon et le détecteur était positionné à 360 mm. Les diagrammes de rayons X étaient par conséquent enregistrés pour une plage d'écartement réciproque q = $4\pi^*\sin \theta/\lambda$ de 0,03-1,8 Å$^{-1}$, où $\theta$ est l'angle de diffraction. Les distances récurrentes d = $2\pi/q$ devraient se situer entre 200 Å et 3,5 Å. Les échantillons étaient placés dans des capillaires en verre de 1,2-1,3 mm (Glas W. Müller, Allemagne) et introduits dans un dispositif de fixation de capillaires de fabrication maison, qui peut contenir jusqu'à 20 capillaires à une température contrôlée.

**Système tensioactif**

Le système tensioactif utilisé dans une composition conforme à l'invention et permettant d'obtenir la formation d'une phase paracristalline de type lamellaire gel (Lβ) comprend :

i) au moins un tensioactif non ionique de valeur HLB à 25°C inférieure à 8, de préférence le (les) tensioactif(s) de valeur HLB à 25°C inférieure à 8 étant choisi(s) parmi des tensioactifs non ioniques, et
ii) au moins un tensioactif non ionique de valeur HLB à 25°C supérieure ou égale à 8.

Selon un mode de réalisation particulier, une composition selon l'invention comporte un système tensioactif comprenant :

\* au moins un tensioactif non ionique de valeur HLB à 25°C inférieure à 8, et
\* au moins un tensioactif non ionique de valeur HLB à 25°C supérieure ou égale 8,

au moins un parmi le (les) tensioactif(s) non ionique(s) de valeur HLB à 25°C inférieure à 8, et au moins un parmi le (les) tensioactif(s) non ionique(s) de valeur HLB à 25°C supérieure ou égale à 8 répondant à la formule suivante (I) :

$$(ALK-[C(O)]_a-[O]_b)_c-X \qquad (I)$$

Formule (I) dans laquelle :

- ALK est un groupe alkyle en $C_7$-$C_{23}$, de préférence en $C_{11}$-$C_{21}$, plus préférentiellement en $C_{15}$-$C_{17}$,
- a et b des nombres entiers compris entre 0 et 100, c est un nombre entier compris entre 1 et 100, en particulier compris entre 1 et 3, de préférence égal à 1, a et b étant de préférence égal à 0,
- X est un groupe (poly)oxyalkylène éventuellement substitué et/ou terminé par un groupe hydroxy, X étant de préférence un groupe oxyéthylène ($CH_2CH_2O)_n$ ou ($OCH_2CH_2)_n$ dans lequel n est supérieure ou égale à 1, par exemple compris entre 1 et 200, de préférence ledit groupe (poly)oxyalkylène étant un polyéthylène glycol ou étant le résultat d'au moins une substitution d'un groupe hydroxy, de préférence choisi parmi les (poly)glycérols.

Le groupe X est de préférence choisi parmi :

i)

$$HO-(ALK-O)_z-CH2-CH[(OALK)_y-OH]-CH2-(O-ALK)_x-(*)$$

dans laquelle :

- ALK identique ou différent représentant un groupe alkylène en C1-C6, en particulier en C1-C4, de préférence l'éthylène,
- x, y, z étant un entier compris entre 0 et 200, étant entendu que x+y+z différent de 0, de préférence x+y+z étant inclusivement compris entre 1 et 150, en particulier entre 15 et 60 ;

ii)

$$H-(ALK-O)_x-(*) \text{ et } H-(O-ALK)_x-(*),$$

de préférence est $H-(O-ALK)_x-(*)$ dans laquelle :

- ALK identique ou différent représentant un groupe ethylene en C1-C6, en particulier en C1-C4, de préférence l'éthylène,
- x est un entier différent de 0 et de préférence compris entre 1 et 200.

Le (Les) tensioactif(s) non ionique(s) de valeur HLB à 25°C inférieure à 8, et le (les) tensioactif(s) non ionique(s) de valeur HLB à 25°C supérieure ou égale à 8, répondent à la formule (I') suivante :

$$ALK-(O-CH_2-CH_2)_n-OH \qquad (I')$$

Formule (I') dans laquelle :

- ALK est un groupe alkyle en $C_8-C_{24}$, de préférence en $C_{12}-C_{22}$, plus préférentiellement en $C_{16}-C_{18}$,

n étant un entier différent de 0, compris entre 1 et 200, de préférence compris entre 1 et 10, mieux entre 2 et 6 pour le (les) tensioactif(s) non ionique(s) de valeur HLB à 25°C inférieure à 8, et de préférence strictement supérieur à 10, en particulier compris entre 15 et 200, pour le (les) tensioactif(s) non ionique(s) de valeur HLB à 25°C supérieure ou égale à 8.
La valeur HLB (hydrophile-lipophile balance) selon GRIFFIN est définie dans J. Soc. Cosm. Chem. 1954 (volume 5), pages 249-256. On peut se reporter au document « Encyclopedia of Chemical Technology, KIRK-OTHMER », volume 22, p. 333-432, 3ème édition, 1979, WILEY, pour la définition des propriétés et des fonctions émulsionnantes des agents tensioactifs, en particulier p. 347-377 de cette référence.
Le (Les) tensioactif(s) non ionique(s) de valeur HLB à 25°C inférieure à 8, de préférence répondant à la formule (I), est (sont) présent(s) à une teneur supérieure ou égale à 5 % en poids par rapport au poids total de la composition, de préférence compris entre 7 et 30%, de préférence compris entre 10 et 20% en poids par rapport au poids total de la composition.
Le (Les) tensioactif(s) non ionique(s) de valeur HLB à 25°C supérieure ou égale à 8, de préférence supérieure ou égale à 10, de préférence répondant à la formule (I), est (sont) présent(s) à une teneur supérieure ou égale à 5 % en poids par rapport au poids total de la composition, de préférence compris entre 7 et 30%, de préférence compris entre 10 et 20% en poids par rapport au poids total de la composition.
Le (Les) tensioactif(s) non ionique(s) de valeur HLB à 25°C inférieure à 8 et le (les) tensioactif(s) non ionique(s) de valeur HLB à 25°C supérieure ou égale à 8, répondant de préférence tous deux à la formule (I), sont présents à une teneur totale supérieure ou égale à 15 %, en particulier comprise entre 16 et 40% en poids, mieux entre 18 et 30% en poids, par rapport au poids total de la composition.
Le (Les) tensioactif(s) non ionique(s) de valeur HLB à 25°C inférieure à 8 et le (les) tensioactif(s) non ionique(s) de valeur HLB à 25°C supérieure ou égale à 8, répondant de préférence tous deux à la formule (I), sont présents à une teneur totale respective telle que le rapport pondéral du (des) tensioactif(s) non ionique(s) de valeur HLB à 25°C inférieure à 8 sur le (les) tensioactif(s) non ionique(s) de valeur HLB à 25°C supérieure ou égale à 8 va de 1/5 à 5, de préférence de 1/3 à 3, de préférence de 2/3 à 3/2.

*Tensioactif(s) non ionique(s) de valeur HLB à 25°C inférieure à 8*

Le (les) tensioactif(s) non ionique(s) de valeur HLB, au sens de Griffin, à 25°C, inférieure à 8 peuvent avantageusement être choisi(s) parmi :

- les esters et éthers d'oses éventuellement (poly)oxyalkylénés, de préférence (poly)oxyalkylénés ;
- les esters d'acides gras, notamment en $C_8$-$C_{24}$, et de préférence en $C_{16}$-$C_{22}$, et de polyol éventuellement (poly)oxyalkyléné, de préférence (poly)oxyalkyléné, notamment de glycérol (poly)oxyalkyléné ou de sorbitol (poly)oxyalkyléné, de préférence de glycérol (poly)oxyalkyléné ;
- les alcools éventuellement (poly)oxyalkylénés, de préférence (poly)oxyalkylénés ;
- et leurs mélanges ; de préférence parmi les alcools éventuellement (poly)oxyalkylénés, de préférence (poly)oxyalkylénés comportant de préférence de 1 à 10 motifs oxyéthylènes.

Par (poly)oxyalkylénés, on entend de 1 à 10 groupe(s) (ou motifs) oxyéthylène(s), mieux de 2 à 6 groupe(s) oxyéthylène(s).

Le (les) au moins un parmi le (les) tensioactif(s) non ionique(s) de valeur HLB à 25°C inférieure à 8 comprend de préférence un alcool éventuellement (poly)oxyalkyléné, de préférence (poly)oxyalkyléné comprenant un éther d'alcool gras en $C_8$-$C_{24}$ et de polyéthylène glycol, ledit éther comprenant de 1 à 10, mieux entre 2 et 6, motifs d'éthylène glycol.

*Tensioactif(s) non ionique(s) de valeur HLB à 25°C supérieure ou égale à 8*

Le (les) tensioactif(s) non ionique(s) de valeur HLB, au sens de Griffin, à 25°C, supérieure ou égale à 8 peuvent avantageusement être choisi(s) parmi :

- les éthers de glycérol (poly)oxyalkyléné, en particulier oxyéthylénés et/ou oxypropylénés, pouvant comporter plus de 10 motifs oxyéthylène et/ou oxypropylène, en particulier de 15 à 200 motifs, mieux de 15 à 100 motifs oxyéthylène et/ou oxypropylène ;
- les alcools (poly)oxyalkylénés, en particulier oxyéthylénés et/ou oxypropylénés, pouvant comporter plus de 10 motifs oxyéthylène et/ou oxypropylène, en particulier de 15 à 200 motifs, mieux de 15 à 100 motifs oxyéthylène et/ou oxypropylène, en particulier les alcools gras, notamment en $C_8$-$C_{24}$, et de préférence en $C_{12}$-$C_{18}$, éthoxylés tels que l'alcool stéarylique éthoxylé à 20 motifs oxyéthylène (nom CTFA "Steareth-20 ") comme le BRIJ 78 commercialisé par la société UNIQEMA, l'alcool cétéarylique éthoxylé à 30 motifs oxyéthylène (nom CTFA "Ceteareth-30 ") ;
- les esters d'acide gras (poly)oxyalkyléné, en particulier les esters d'acide gras, notamment en $C_8$-$C_{24}$, et de préférence en $C_{16}$-$C_{22}$, et de polyéthylène glycol (ou PEG) (pouvant comprendre plus de 10 motifs oxyéthylène, en particulier de 15 à 200 motifs, mieux de 15 à 100 motifs oxyéthylène), tels que le stéarate de PEG-50 et le monostéarate de PEG-40 commercialisé sous le nom MYRJ 52P® par la société UNIQEMA;
- les esters d'acide gras, notamment en $C_8$-$C_{24}$, et de préférence en $C_{16}$-$C_{22}$, et d'éthers de glycérol (poly)oxyalkyléné, en particulier oxyéthylénés et/ou oxypropylénés (pouvant comporter plus de 10 motifs oxyéthylène et/ou oxypropylène, en particulier de 15 à 200 motifs, mieux de 15 à 100 motifs oxyéthylène et/ou oxypropylène), comme le monostéarate de glycéryle polyoxyéthyléné à 200 motifs oxyéthylène, vendu sous la dénomination Simulsol 220 TM® par la société SEPPIC ; le stéarate de glycéryle polyoxyéthyléné à 30 motifs oxyéthylène comme le produit TAGAT S® vendu par la société GOLDSCHMIDT, l'oléate de glycéryle polyoxyéthyléné à 30 motifs oxyéthylène comme le produit TAGAT O® vendu par la société GOLDSCHMIDT, le cocoate de glycéryle polyoxyéthyléné à 30 motifs oxyéthylène comme le produit VARIONIC LI 13® vendu par la société SHEREX, l'isostéarate de glycéryle polyoxyéthyléné à 30 motifs oxyéthylène comme le produit TAGAT L® vendu par la société GOLDSCHMIDT et le laurate de glycéryle polyoxyéthyléné à 30 motifs oxyéthylène comme le produit TAGAT I® de la société GOLDSCHMIDT ;
- les esters d'acide gras, notamment en $C_8$-$C_{24}$, et de préférence en $C_{16}$-$C_{22}$, et d'éthers de sorbitol (poly)oxyalkyléné, en particulier oxyéthylénés et/ou oxypropylénés (pouvant comporter plus de 10 motifs oxyéthylène et/ou oxypropylène, en particulier de 15 à 200 motifs, mieux de 15 à 100 motifs oxyéthylène et/ou oxypropylène), comme le polysorbate 60 vendu sous la dénomination Tween 60® par la société UNIQEMA ;
- et leur(s) mélange(s) ; de préférence parmi les alcools (poly)oxyalkylénés comportant de préférence plus de 10 motifs oxyéthylène, en particulier de 15 à 200 motifs, mieux de 15 à 100 motifs oxyéthylène (ou d'éthylène glycol).

De préférence, une composition comporte au moins un tensioactif(s) non ionique(s) de valeur HLB, au sens de Griffin, à 25°C, supérieure ou égale à 8, de préférence supérieure ou égale à 10, choisi parmi au moins un éther d'alcool gras en $C_8$-$C_{24}$, de préférence en $C_{12}$-$C_{22}$ plus préférentiellement en $C_{16}$-$C_{18}$, et de polyéthylène glycol, ledit éther comprenant plus de 10 motifs d'éthylène glycol, mieux entre 15 et 200 motifs d'éthylène glycol .

De préférence, une composition conforme à l'invention est exempte de phosphates d'alkyle et en particulier est exempte de phosphate de cétyle.

De préférence, une composition conforme à l'invention est exempte de tensioactif(s) amphotère(s).

Par ailleurs le système tensioactif peut comprendre un ou plusieurs co-tensioactif(s) choisis parmi les alcools gras comprenant de 10 à 26 atomes de carbone, mieux de 12 à 24 atomes de carbone et encore mieux de 14 à 22 atomes de carbone. Toutefois, ce(s) co-tensioactif(s) n'interviennent pas dans le calcul de la teneur totale en système tensioactif conforme à l'invention.

### *Polymère(s) filmogène(s)*

La composition selon l'invention comprend au moins une dispersion aqueuse de particules de polymère(s) filmogène(s) et éventuellement au moins un polymère filmogène additionnelle (non présent sous forme de dispersion aqueuse de particules, tel qu'un polymère filmogène hydrosoluble).

Dans la présente demande, on entend par "polymère filmogène", un polymère apte à former à lui seul ou en présence d'un agent auxiliaire de filmification, un dépôt macroscopiquement continu, et de préférence un dépôt cohésif, et mieux encore un dépôt dont la cohésion et les propriétés mécaniques sont telles que ledit dépôt peut être isolable et manipulable isolément, par exemple lorsque ledit dépôt est réalisé par coulage sur une surface anti-adhérente comme une surface téflonnée ou siliconée.

Une composition selon l'invention comporte de préférence une teneur totale en matière sèche de polymère(s) filmogène(s) supérieure ou égale à 5% en poids, de préférence à 10% en poids, par rapport au poids total de la composition, mieux à 12% en poids, par rapport au poids total de la composition.

Une composition selon l'invention comporte de préférence une teneur totale en matière sèche de polymère(s) filmogène(s) allant de 10 à 30 % en poids par rapport au poids total de la composition, mieux de 12 à 25 %.

La composition selon l'invention comprend de préférence plus précisément au moins une dispersion aqueuse de particules formées d'un ou plusieurs polymères filmogènes.

La teneur totale en particules de polymère(s) filmogène(s) et la teneur totale en système tensioactif sont avantageusement telles que le rapport pondéral des particules de polymère(s) filmogène(s) / le système tensioactif conforme à l'invention est inférieur ou égal à 1.75, de préférence inférieur ou égal à 1.5, en particulier compris entre 1/2 et 1.25

Elle peut également comporter au moins un polymère filmogène hydrosoluble. Ainsi une composition peut comporter au moins un polymère filmogène additionnel, distinct de particules de polymère(s) filmogène(s) présente(s) sous forme de dispersion aqueuse. La teneur en ce (ces) polymère(s) filmogène(s) additionnel(s), dit hydrosoluble(s), est de préférence inférieure ou égale 10 % en poids par rapport au poids total de la composition, plus préférentiellement encore à 5 % en poids, mieux à 2% en poids, par rapport au poids total de la composition.

### Polymère(s) filmogène(s) en dispersion aqueuse

Un tel polymère filmogène présent dans ladite préparation de la composition sous la forme de particules en dispersion aqueuse, portent généralement le nom de (pseudo)latex, c'est-à-dire latex ou pseudolatex. Les techniques de préparation de ces dispersions sont bien connues de l'homme du métier.

Une dispersion convenant à l'invention, peut comprendre un ou plusieurs types de particules, ces particules pouvant varier par leur taille, par leur structure et/ou par leur nature chimique.

Une composition selon l'invention comporte une teneur totale en matière sèche de particules de polymère(s) filmogène(s) sous forme de dispersion aqueuse supérieure ou égale à 5 % en poids par rapport au poids total de la composition.

Avantageusement, une composition selon l'invention comporte une teneur totale en matière sèche de particules de polymère(s) filmogène(s) sous forme de dispersion aqueuse supérieure ou égale à 10% en poids, voire à 12 % en poids, par rapport au poids total de la composition.

Une composition selon l'invention comporte de préférence une teneur totale en matière sèche de particules de polymère(s) filmogène(s) allant de 10 à 30 % en poids par rapport au poids total de la composition, mieux de 12 à 25 % en poids.

La teneur totale en particules de polymère(s) filmogène(s) présente(s) sous forme de dispersion(s) aqueuse(s) est de préférence supérieure ou égale à 30 % en poids, préférentiellement à 40 % en poids, par rapport au poids total des particules.

Ces particules peuvent être de nature anionique, cationique ou neutre et peuvent constituer un mélange de particules de natures différentes.

Parmi les polymères filmogènes utilisables dans la composition de la présente invention, on peut citer les polymères synthétiques, de type radicalaire ou de type polycondensat, les polymères d'origine naturelle et leurs mélanges. D'une manière générale ces polymères peuvent être des polymères statistiques, des copolymères blocs de type A-B, multi blocs A- B-A ou encore ABCD..., voire des polymères greffés.

*Polymère filmogène radicalaire*

Par « polymère radicalaire », on entend un polymère obtenu par polymérisation de monomères à insaturation notamment éthylénique, chaque monomère étant susceptible de s'homopolymériser (à l'inverse des polycondensats).

Les polymères filmogènes de type radicalaire peuvent être notamment des homopolymères ou des copolymères, acryliques et /ou vinyliques.

Les polymères filmogènes vinyliques peuvent résulter de la polymérisation de monomères à insaturation éthylénique ayant au moins un groupement acide et/ou des esters de ces monomères acides et/ou des amides de ces monomères acides.

Comme monomères à insaturation éthylénique ayant au moins un groupement acide ou monomère porteur de groupement acide, on peut utiliser des acides carboxyliques insaturés $\alpha,\beta$-éthyléniques tels que l'acide acrylique, l'acide méthacrylique, l'acide crotonique, l'acide maléique, l'acide itaconique. On utilise en particulier l'acide (méth)acrylique et l'acide crotonique, et plus particulièrement l'acide (méth)acrylique.

Les esters de monomères acides sont avantageusement choisis parmi les esters de l'acide (méth)acrylique (encore appelé les (méth)acrylates), notamment des (méth)acrylates d'alkyle, en particulier d'alkyle en $C_1$-$C_{20}$, plus particulièrement en $C_1$-$C_8$, des (méth)acrylates d'aryle, en particulier d'aryle en $C_6$-$C_{10}$, des (méth)acrylates d'hydroxyalkyle, en particulier d'hydroxyalkyle en $C_2$-$C_6$.

Parmi les (méth)acrylates d'alkyle, on peut citer le méthacrylate de méthyle, le méthacrylate d'éthyle, le méthacrylate de butyle, le méthacrylate d'isobutyle, le méthacrylate d'éthyl-2 hexyle, le méthacrylate de lauryle.

Parmi les (méth)acrylates d'hydroxyalkyle, on peut citer l'acrylate d'hydroxyéthyle, l'acrylate de 2-hydroxypropyle, le méthacrylate d'hydroxyéthyle, le méthacrylate de 2-hydroxypropyle.

Parmi les (méth)acrylates d'aryle, on peut citer l'acrylate de benzyle et l'acrylate de phényle.

Les esters de l'acide (méth)acrylique sont en particulier des (méth)acrylates d'alkyle.

Selon la présente invention, le groupement alkyle des esters peut être soit fluoré, soit perfluoré, c'est-à-dire qu'une partie ou la totalité des atomes d'hydrogène du groupement alkyle sont substitués par des atomes de fluor.

Comme amides des monomères acide, on peut par exemple citer les (méth)acrylamides, et notamment les N-alkyl (méth)acrylamides, en particulier d'alkyle en $C_2$-$C_{12}$. Parmi les N-alkyl (méth)acrylamides, on peut citer le N-éthyl acrylamide, le N-t-butyl acrylamide et le N-t-octyl acrylamide.

Les polymères filmogènes vinyliques peuvent également résulter de l'homopolymérisation ou de la copolymérisation de monomères choisis parmi les esters vinyliques et les monomères styrèniques. En particulier, ces monomères peuvent être polymérisés avec des monomères acides et/ou leurs esters et/ou leurs amides, tels que ceux mentionnés précédemment.

Comme exemple d'esters vinyliques, on peut citer l'acétate de vinyle, le néodécanoate de vinyle, le pivalate de vinyle, le benzoate de vinyle et le t-butyl benzoate de vinyle.

Comme monomères styrèniques, on peut citer le styrène de l'alpha-méthyl styrène.

La liste des monomères donnée n'est pas limitative et il est possible d'utiliser tout monomère connu de l'homme du métier entrant dans les catégories de monomères acryliques et vinyliques (y compris les monomères modifiés par une chaîne siliconée).

Comme polymère vinylique, on peut également utiliser les polymères acryliques siliconés.

On peut également citer les polymères résultant de la polymérisation radicalaire d'un ou plusieurs monomères radicalaires, à l'intérieur et/ou partiellement à la surface de particules préexistantes d'au moins un polymère choisi parmi le groupe constitué par les polyuréthanes, les polyurées, les polyesters, les polyesteramides et/ou les alkydes. Ces polymères sont généralement appelés « polymères hybrides ».

*Polycondensat*

Comme polymère filmogène de type polycondensat, on peut citer les polyuréthannes anioniques, cationiques, non-ioniques ou amphotères, les polyuréthannes-acryliques, les polyuréthannes-polyvinylpyrrolidones, les polyester-polyuréthannes, les polyéther-polyuréthannes, les polyurées, les polyurée/polyuréthannes, les polyuréthannes siliconés, et leurs mélanges.

Le polyuréthanne filmogène peut être, par exemple, un copolymère polyuréthanne, polyurée/uréthanne, ou polyurée, aliphatique, cycloaliphatique ou aromatique, comportant seule ou en mélange au moins une séquence choisie parmi :

- une séquence d'origine polyester aliphatique et/ou cycloaliphatique et/ou aromatique, et/ou,

- une séquence siliconée, ramifiée ou non, par exemple polydiméthylsiloxane ou polyméthylphénylsiloxane, et/ou
- une séquence comportant des groupes fluorés.

Les polyuréthannes filmogènes tels que définis dans l'invention peuvent être également obtenus à partir de polyesters, ramifiés ou non, ou d'alkydes comportant des hydrogènes mobiles que l'on modifie par réaction avec un diisocyanate et un composé organique bifonctionnel (par exemple dihydro, diamino ou hydroxyamino), comportant en plus soit un groupement acide carboxylique ou carboxylate, soit un groupement acide sulfonique ou sulfonate, soit encore un groupement amine tertiaire neutralisable ou un groupement ammonium quaternaire.

Parmi les polycondensats filmogènes, on peut également citer les polyesters, les polyesters amides, les polyesters à chaîne grasse, les polyamides, et les résines époxyesters.

Les polyesters peuvent être obtenus, de façon connue, par polycondensation d'acides dicarboxyliques avec des polyols, notamment des diols.

L'acide dicarboxylique peut être aliphatique, alicyclique ou aromatique. On peut citer comme exemple de tels acides : l'acide oxalique, l'acide malonique, l'acide diméthylmalonique, l'acide succinique, l'acide glutarique, l'acide adipique, l'acide pimélique, l'acide 2,2-diméthylgluratique, l'acide azélaïque, l'acide subérique, l'acide sébacique, l'acide fumarique, l'acide maléique, l'acide itaconique, l'acide phtalique, l'acide dodécanedioïque, l'acide 1,3-cyclohexanedicarbocylique, l'acide 1,4-cyclohexanedicarboxylique, l'acide isophtalique, l'acide téréphtalique, l'acide 2,5-norborane dicarboxylique, l'acide diglycolique, l'acide thiodipropionique, l'acide 2,5-naphtalènedicarboxylique, l'acide 2,6-naphtalène-dicarboxylique. Ces monomères acide dicarboxylique peuvent être utilisés seuls ou en combinaison d'au moins deux monomères acide dicarboxylique. Parmi ces monomères, on choisit en particulier l'acide phtalique, l'acide isophtalique et l'acide téréphtalique.

Le diol peut être choisi parmi les diols aliphatiques, alicycliques, aromatiques. On utilise en particulier un diol choisi parmi : l'éthylène glycol, le diéthylène glycol, le triéthylène glycol, le 1,3-propanediol, le cyclohexane diméthanol, le 4-butanediol. Comme autres polyols, on peut utiliser le glycérol, le pentaérythritol, le sorbitol, le triméthylol propane.

Les polyesters amides peuvent être obtenus de manière analogue aux polyesters, par polycondensation de diacides avec des diamines ou des amino alcools. Comme diamine, on peut utiliser l'éthylènediamine, l'hexaméthylènediamine, la méta- ou para-phénylènediamine. Comme aminoalcool, on peut utiliser la monoéthanolamine.

*Polymère d'origine naturelle*

On peut utiliser dans la présente invention des polymères d'origine naturelle, éventuellement modifiés, comme la résine shellac, la gomme de sandaraque, les dammars, les élémis, les copals, les polymères cellulosiques insolubles dans l'eau tels que la nitrocellulose, les esters de cellulose modifiés dont notamment des esters de carboxyalkyl cellulose comme ceux décrits dans la demande de brevet US 2003/185774, et leurs mélanges.

Selon un mode de réalisation particulier de l'invention, ledit au moins un polymère filmogène à l'état dispersé est choisi parmi les dispersions de polymères acryliques, les dispersions de polyuréthanne, les dispersions de sulfopolyesters, les dispersions vinyliques, les dispersions de polyvinyl acétate, les dispersions de terpolymère vinyl pyrrolidone, diméthylaminopropyl méthacrylamide et chlorure de lauryldiméthylpropylméthacrylamidoammonium, les dispersions de polymère hybrides polyuréthane/polyacryliques, les dispersions de particules de type cœur-écorce et leurs mélanges.

Sont détaillés ci-après différents types de dispersions aqueuses, notamment commerciales, adaptées à la préparation de la composition conforme à la présente invention.

**1/** Ainsi, selon un mode de réalisation préféré de l'invention, la dispersion aqueuse de particules de polymère est une dispersion aqueuse de polymère acrylique.

Le polymère acrylique peut être un copolymère styrène/acrylate, et notamment un polymère choisi parmi les copolymères issus de la polymérisation d'au moins un monomère styrénique et au moins un monomère (méth)acrylate d'alkyle en $C_1$-$C_{18}$.

Comme monomère styrénique utilisable dans l'invention, on peut citer par exemple le styrène ou l'alpha-méthylstyrène, et en particulier le styrène.

Le monomère de (méth)acrylate d'alkyle en $C_1$-$C_{18}$ est en particulier un (méth)acrylate d'alkyle en $C_1$-$C_{12}$ et plus particulièrement un (méth)acrylate d'alkyle en $C_1$-$C_{10}$. Le monomère (méth)acrylate d'alkyle en $C_1$-$C_{18}$ peut être choisi parmi l'acrylate de méthyle, le méthacrylate de méthyle, l'acrylate d'éthyle, l'acrylate de propyle, l'acrylate de butyle, le méthacrylate de butyle, l'acrylate d'hexyle, l'acrylate d'octyle, l'acrylate de 2-éthyl hexyle, le (méth)acrylate de lauryle et le (méth)acrylate de stéaryle.

Comme polymère acrylique en dispersion aqueuse, on peut utiliser selon l'invention le copolymère styrène/acrylate commercialisé sous la dénomination « Joncryl SCX-8211® » par la société BASF ou « SYNTRAN 5760CG » par la société Interpolymer, le polymère acrylique commercialisé sous la référence « Acronal® DS - 6250 » par la société BASF, le copolymère acrylique « Joncryl® 95 » par la société BASF.

**2/** selon une variante de réalisation de l'invention, la dispersion aqueuse de particules de polymère est une dispersion aqueuse de particules de polyester-polyuréthanne et/ou de polyéther-polyuréthanne en particulier anionique.

Le caractère anionique des polyester-polyuréthannes et des polyéther-polyuréthannes utilisés selon l'invention est du à la présence dans leurs motifs constitutifs de groupements à fonction acide carboxylique ou acide sulfonique.

Les particules de polyester-polyuréthanne ou de polyéther-polyuréthanne utilisées selon l'invention sont généralement commercialisées sous forme de dispersions aqueuses.

La teneur en particules desdites dispersions actuellement disponibles sur le marché, va d'environ 20 % à environ 60 % en poids par rapport au poids total de la dispersion.

Parmi les dispersions de polyester-polyuréthanne anionique utilisables dans les compositions selon l'invention, on peut citer en particulier celle commercialisée sous la dénomination « Avalure UR 405®» par la société NOVEON ou « Baycusan C1004 » par la société BAYER MATERIAL SCIENCE.

Parmi les dispersions de particules de polyéther-polyuréthanne anionique utilisables selon l'invention, on peut citer en particulier celles commercialisées sous la dénomination de « Avalure UR 450® » par la société NOVEON, et sous la dénomination de « Néorez R 970® » par la société DSM.

Selon un mode de réalisation particulier de l'invention, on peut utiliser un mélange de dispersions commerciales constitué de particules de polyester-polyuréthanne anionique telles que définies ci-dessus et de particules de polyéther-polyuréthanne anionique également définies ci-dessus.

Par exemple, on peut utiliser un mélange constitué de la dispersion commercialisée sous la dénomination de « Sancure 861®» ou un mélange de celle commercialisée sous la dénomination de « Avalure UR 405° » et de celle commercialisée sous la dénomination de « Avalure UR 450® », ces dispersions étant commercialisées par la société NOVEON.

**3/** Selon un autre mode de réalisation particulier de l'invention, la dispersion aqueuse utilisé comprend un mélange d'au moins deux polymères filmogènes sous forme de particules distinctes par leurs températures de transition vitreuses (Tg) respectives.

En particulier, selon un mode de réalisation de l'invention, la composition conforme à l'invention peut comprendre au moins un premier polymère filmogène à l'état dispersé et au moins un second polymère filmogène à l'état dispersé, lesdits premier et second polymères ayant des Tg différentes et de préférence la Tg du premier polymère (Tg1) est supérieure à la Tg du second polymère (Tg2). En particulier la différence entre les Tg1 et Tg2 est en valeur absolue, d'au moins 10 °C, de préférence d'au moins 20 °C.

Plus précisément, elle comprend dans un milieu aqueux acceptable :

a) des particules dispersées dans le milieu aqueux d'un premier polymère filmogène ayant au moins une température de transition vitreuse Tg1 supérieure ou égale à 20 °C, et

b) des particules dispersées dans le milieu aqueux d'un deuxième polymère filmogène ayant au moins une température de transition vitreuse Tg2 inférieure ou égale à 70 °C,

Cette dispersion résulte généralement d'un mélange de deux dispersions aqueuses de polymère filmogène.

Le premier polymère filmogène a au moins une, notamment a une température de transition vitreuse Tg1 supérieure ou égale à 20 °C, notamment allant de 20°C à 150 °C, et avantageusement supérieure ou égale à 40 °C, notamment allant de 40 °C à 150 °C, et en particulier supérieure ou égale à 50 °C, notamment allant de 50 °C à 150 °C.

Le deuxième polymère filmogène a au moins une, notamment a une température de transition vitreuse Tg2 inférieure ou égale à 70 °C, notamment allant de -120 °C à 70 °C, et en particulier inférieure à 50 °C, notamment allant de -60 °C à +50 °C, et plus particulièrement allant de -30 °C à 30 °C.

La mesure de la température de transition vitreuse (Tg) d'un polymère est effectuée par DMTA (Dynamical and Mechanical Temperature Analysis ou Analyse dynamique et mécanique de température) comme décrit ci-dessous.

Pour mesurer la température de transition vitreuse (Tg) d'un polymère, on effectue des essais de viscoélasticimétrie avec un appareil DMTA de « Polymer laboratories », sur un échantillon de film. Ce film est préparé par coulage de la dispersion aqueuse de polymère filmogène dans une matrice téflonnée puis séché à 120 °C pendant 24 heures. On obtient alors un film dans lequel on découpe (par exemple à l'emporte-pièce) les éprouvettes. Celles-ci ont typiquement une épaisseur d'environ 150 $\mu$m, une largeur de 5 à 10 mm et une longueur utile d'environ 10 à 15 mm. On impose à cet échantillon une sollicitation de traction. L'échantillon subit une force statique de 0,01 N à laquelle se superpose un déplacement sinusoïdal de $\pm$ 8 $\mu$m à la fréquence de 1 Hz. On travaille ainsi dans le domaine linéaire, sous de faibles niveaux de déformation. Cette sollicitation de traction est effectuée sur l'échantillon à des températures variant de -150 °C à +200 °C, avec une variation de température de 3 °C par minute.

On mesure alors le module complexe $E^* = E' + iE''$ du polymère testé en fonction de la température.

De ces mesures, on déduit les modules dynamiques E', E'' et le pouvoir amortissant : $tg\delta = E''/E'$.

Puis on trace la courbe des valeurs de $tg\delta$ en fonction de la température ; cette courbe présente au moins un pic. La température de transition vitreuse Tg du polymère correspond à la température à laquelle se situe le sommet de ce pic.

Lorsque la courbe présente au moins 2 pics (dans ce cas, le polymère présente au moins 2 Tg), on prend comme valeur de Tg du polymère testé la température pour laquelle la courbe présente un pic de plus forte amplitude (c'est-à-dire correspondant à la plus grande valeur de tg$\delta$ ; on ne considère dans ce cas que la Tg « majoritaire » comme valeur de Tg du polymère testé).

Dans la présente invention, la température de transition Tg1 correspond à la Tg « majoritaire » (au sens défini précédemment) du premier polymère filmogène lorsque ce dernier présente au moins 2 Tg ; la température de transition vitreuse Tg2 correspond à la Tg « majoritaire » du deuxième polymère filmogène lorsque ce dernier présente au moins 2 Tg.

Le premier polymère filmogène et le deuxième polymère filmogène peuvent être choisis, indépendamment l'un de l'autre, parmi les polymères radicalaires, les polycondensats et les polymères d'origine naturelle tels que définis précédemment ayant les caractéristiques de température de transition vitreuse définies précédemment.

Comme premier polymère filmogène en dispersion aqueuse, on peut utiliser les dispersions aqueuses de polymère vendues sous les dénominations « NeoRez R-989® » par la société DSM, « Joncryl 95 » et « Joncryl® 8211 » par la société BASF.

Comme deuxième polymère filmogène en dispersion aqueuse, on peut utiliser par exemple les dispersions aqueuses de polymère vendues sous les dénominations « Avalure® UR-405 », « Avalure® UR-460 » par la société NOVEON ou « Acrilem IC89RT® » par la société ICAP, Néocryl A-45 par la société DSM.

Le polymère filmogène de la dispersion aqueuse « Avalure® UR-460 » est un polyuréthanne obtenu par la polycondensation de polyoxyde de tétraméthylène, de diisocyanate de tétraméthylxylylène, de diisocyanate d'isophorone et d'acide diméthylol propionique.

Selon un mode de réalisation tout particulièrement préféré de l'invention, on utilise comme premier et second polymères filmogènes en dispersion aqueuse l'association de dispersion de polymère styrène/acrylate telle que la dispersion commercialisée sous la référence « Joncryl 8211® » par BASF et de dispersion de polymère acrylique telle que la dispersion commercialisée sous la référence « Neocryl A-45® » par DSM.

Selon un autre mode de réalisation préféré de ce mode de réalisation particulier du point 3/ ci-dessus de l'invention, on utilise comme premier polymère filmogène en dispersion aqueuse une dispersion de polymère acrylique telle que la dispersion commercialisée sous la référence « Joncryl 95® » par BASF et comme second polymère filmogène une dispersion de polymère polyuréthane anionique commercialisée sous la référence « Avalure UR405® » par DSM.

Comme dispersions aqueuses de polymère filmogène, on peut utiliser :

- les dispersions acryliques vendues sous les dénominations « Acronal DS-6250® » par la société BASF, « Neocryl A-45® », « Neocryl XK-90® », « Neocryl A-1070® », « Neocryl A-1090® », « Neocryl BT-62® », « Neocryl A-1079® » et « Neocryl A-523® » par la société DSM, « Joncryl 95® », « Joncryl 8211® » par la société BASF, « Daitosol 5000 AD® » ou « Daitosol 5000 SJ » par la société DAITO KASEY KOGYO ; « Syntran 5760 CG» par la société Interpolymer,
- les dispersions aqueuses de polyuréthanne vendues sous les dénominations « Neorez R-981® » et « Neorez R-974® » par la société DSM, les « Avalure UR-405® », « Avalure UR-410® », « Avalure UR-425® », « Avalure UR-450® », « Sancure 875® », « Avalure UR 445® », « Avalure UR 450® » par la société NOVEON, « Impranil 85® » par la société BAYER, « Baycusan C1004® » par la société BAYER MATERIAL SCIENCE,
- les sulfopolyesters vendus sous le nom de marque « Eastman AQ® » par la société EASTMAN CHEMICAL PRODUCTS,
- les dispersions vinyliques comme le « Mexomère PAM », les dispersions aqueuses de polyvinyl acétate comme le « Vinybran® » de la société Nisshin Chemical ou celles commercialisées par la société UNION CARBIDE, les dispersions aqueuses de terpolymère vinyl pyrrolidone, diméthylaminopropyl méthacrylamide et chlorure de lauryldiméthylpropylméthacrylamidoammonium telles que le « Styleze W® »-d'ISP,
- les dispersions aqueuses de polymère hybrides polyuréthane/polyacryliques telles que celles commercialisées sous les références « Hybridur® » par la société AIR PRODUCTS ou « Duromer® » de NATIONAL STARCH,
- les dispersions de particules de type cœur-écorce telles que celles commercialisées par la société ARKEMA sous la référence « Kynar® » (coeur : fluoré - écorce : acrylique) ou encore celles décrites dans US 5 188 899 (coeur : silice - écorce : silicone) et leurs mélanges.

Selon un mode de réalisation préféré, une composition conforme à l'invention comprend une dispersion aqueuse de particules choisies parmi les dispersions aqueuse de polymère(s) filmogène(s) acryliques et dérivés, en particulier de styrène-acrylique et dérivés, et les dispersions aqueuse de polymère(s) polyuréthane(s), en particulier de polyester-polyuréthanne, et leurs dérivés, et leur(s) mélange.

*Polymère filmogène hydrosoluble*

Les compositions selon la présente invention comportent au moins un polymère filmogène hydrosoluble.

De façon préférée, une composition selon l'invention est exempte de polymère filmogène hydrosoluble. Toutefois, la teneur totale en matière sèche de "polymère(s) filmogène(s) hydrosoluble(s)" peut aller de 0,1 à 10 %, de préférence de 0,5 à 8 % et mieux de 1 à 5 % en poids, par rapport au poids total de la composition.

Comme exemples de polymères filmogènes hydrosolubles, on peut citer :

- les protéines comme les protéines d'origine végétale telles que les protéines de blé, de soja ; les protéines d'origine animale tels que les kératines, par exemples les hydrolysats de kératine et les kératines sulfoniques ;
- les polymères de cellulose tels que l'hydroxyéthylcellulose, l'hydroxypropylcellulose, la méthylcellulose, l'éthylhydroxyéthylcellulose, la carboxyméthylcellulose, ainsi que les dérivés quaternisés de la cellulose ;
- les polymères ou copolymères acryliques, tels que les polyacrylates ou les polyméthacrylates ;
- les polymères vinyliques, comme les polyvinylpyrrolidones, les copolymères de l'éther méthylvinylique et de l'anhydride malique, le copolymère de l'acétate de vinyle et de l'acide crotonique, les copolymères de vinylpyrrolidone et d'acétate de vinyle ; les copolymères de vinylpyrrolidone et de caprolactame ; l'alcool polyvinylique ;
- les polymères de chitine ou de chitosane anioniques, cationiques, amphotères ou non-ioniques ,
- les gommes arabiques, la gomme de guar, les dérivés du xanthane, la gomme de karaya, la gomme d'acacia ;
- les alginates et les carraghénanes ;
- les glycoaminoglycanes, l'acide hyaluronique et ses dérivés ;
- l'acide désoxyribonucléïque ;
- les muccopolysaccharides tels les chondroïtines sulfate,

et leurs mélanges.

## Charges

Une composition selon l'invention peut comprendre au moins une charge.

Par « charges », il faut comprendre les particules incolores ou blanches, solides, de toutes formes, qui se présentent sous une forme insoluble et dispersée dans le milieu de la composition. De nature minérale ou organique, elles permettent de conférer à la composition de la douceur, de la matité et de l'uniformité au maquillage.

Les charges peuvent être ou non enrobées superficiellement, et, en particulier, elles peuvent être traitées en surface par des silicones, des acides aminés, des dérivés fluorés ou toute autre substance favorisant la dispersion et la compatibilité de la charge dans la composition.

Une composition selon l'invention possède avantageusement une teneur en charges supérieure ou égale à 5 % en poids, mieux à 8% en poids, par rapport au poids total de la composition, avantageusement comprise entre 8 et 30% en poids, mieux entre 8 et 25% en poids, mieux entre 10 et 20% en poids, par rapport au poids total de la composition.

La composition peut comprendre au moins une charge choisie parmi les charges sphériques, les charges lamellaires et leur(s) mélange(s).

*Charges sphériques*

Par « charges sphériques», il faut comprendre les charges comprenant au moins une portion générale arrondie, de préférence définissant au moins une portion de sphère, de préférence définissant intérieurement une concavité ou un creux.

De telles charges dites sphériques peuvent être des charges parfaitement sphériques, des charges globulaires, des charges hémi-sphériques, des charges en forme de bol ou encore des charges en forme de fer-à-cheval.

La (Les) charge(s) sphérique(s) sont de préférence creuse, étant le cas échéant aptes à absorber et/ou adsorbe au moins en partie la phase huileuse, et plus généralement la phase grasse.

La (les) charge(s) sphérique(s) conforme(s) à l'invention est (sont) avantageusement une (des) particule(s) absorbant le sébum, ayant une prise de sébum. On entend par « particule absorbant le sébum » une poudre apte à absorber et/ou adsorber le sébum.

La prise de sébum correspond à la quantité de sébum absorbé et/ou adsorbé par la particule. Elle est mesurée selon la méthode du Wet Point comme suit.

Méthode de mesure de prise de sébum d'une poudre :

La prise de sébum d'une poudre est mesurée selon la méthode de détermination de prise d'huile de poudre décrite dans la norme NF T 30-022. Elle correspond à la quantité de sébum adsorbé sur la surface disponible de la poudre par mesure du Wet Point.

On place une quantité m (en grammes) de poudre comprise entre environ 0,5 g et 5 g (la quantité dépend de la densité de la poudre) sur une plaque de verre puis on ajoute goutte à goutte du sébum artificiel ayant la composition suivante :

| | |
|---|---|
| - trioléine | 29 % |
| - acide oléïque | 28,5 % |
| - oléate d'oléyle | 18,5 % |
| - squalène | 14 % |
| - cholestérol | 7 % |
| - palmitate de cholestérol | 3 % |

Après addition de 4 à 5 gouttes de sébum artificiel, on incorpore le sébum artificiel dans la poudre à l'aide d'une spatule et on continue d'ajouter du sébum artificiel jusqu'à la formation de conglomérats de sébum artificiel et de poudre. A partir de ce moment, on ajoute le sébum artificiel à raison d'une goutte à la fois et on triture ensuite le mélange avec la spatule. On cesse l'addition de sébum artificiel lorsque l'on obtient une pâte ferme et lisse. Cette pâte doit se laisser étendre sur la plaque de verre sans craquelures ni formation de grumeaux. On note alors le volume Vs (exprimé en ml) de sébum artificiel utilisé.

La prise de sébum correspond au rapport Vs / m.

Avantageusement, les charges sphériques conformes à l'invention ont une prise de sébum supérieure ou égale à 10 ml/100g, notamment supérieure ou égale à 20 ml/100g, et en particulier supérieure ou égale à 30 ml/100g, de préférence supérieure ou égale à 40 ml/100g, et en particulier compris inclusivement entre 45 et 1500 ml/100g, ou encore entre 45 et 300 ml/100g.

La (Les) charge(s) sphérique(s) ont avantageusement un diamètre moyen, encore appelé diamètre médian ou taille moyenne en nombre, indiqué par une valeur $D_{50}$, va de 0,05 $\mu$m à 50 $\mu$m, de préférence va de 2 à 40 $\mu$m. Cette dimension $D_{50}$ est donnée par la distribution granulométrique statistique à la moitié de la population, dite D50

La (Les) charge(s) sphérique(s) est (sont) présente(s) à une teneur totale supérieure ou égale à 5 % en poids, par rapport au poids total de la composition, par exemple compris entre 8 et 30% en poids, par rapport au poids total de la composition, de préférence compris entre 8 et 25% en poids, mieux entre 10 et 20% en poids,;

La (Les) charge(s) sphérique(s) et le système tensioactif conforme à l'invention sont avantageusement présents à une teneur pondérale totale respective telle que le rapport pondéral de la (des) charge(s) sphérique(s) sur le système tensioactif est supérieur ou égal à 1/10, de préférence compris entre 1/5 et 6/5 ;

La (les) charge(s) sphérique(s) et le (les) tensioactif(s) non ionique(s) de valeur HLB supérieure ou égale à 8 sont avantageusement présents dans la composition dans une teneur totale respective telle que le rapport pondéral de la (des) charge(s) sphérique(s) sur le (les) tensioactif(s) non ionique(s) de valeur HLB supérieure ou égale à 8 va de 1/20 à 1, de préférence de 1/10 à 2/3.

La (les) charge(s) sphérique(s) et le (les) tensioactif(s) non ionique(s) de valeur HLB inférieure à 8 sont avantageusement présents dans la composition dans une teneur totale respective telle que le rapport pondéral de la (des) charge(s) sphérique(s) sur le (les) tensioactif(s) non ionique(s) de valeur HLB inférieure à 8 va de 1/20 à 1, de préférence de 1/10 à 2/3.

Les charges sphériques peuvent être minérales ou organiques, de préférence organiques.

A titre représentatif et non limitatif de charges selon l'invention, on peut tout particulièrement citer les particules ci-dessous.

Les charges sphériques sont avantageusement choisies parmi :

- des poudres de silice ;
- des poudres de (co)polymères acryliques, et leurs dérivés, en particulier des poudres de (co)polymère acrylate, et leurs dérivés, avantageusement choisies parmi une poudre de polyméthacrylate de méthyle, une poudre de polyméthacrylate de méthyle/diméthacrylate d'éthylène glycol, une poudre de polyméthacrylate d'allyle/diméthacrylate d'éthylène glycol, une poudre de copolymère diméthacrylate d'éthylène glycol/méthacrylate de lauryle, une poudre de copolymère acrylate / alkyl acrylate éventuellement réticulé, des particules creuses de (co-) polymère d'acrylonitrile expansées, et leur(s) mélange(s) ;
- des poudres de polyuréthane ;
- des poudres de silicone avantageusement choisies parmi parmi une poudre de polyméthylsilsesquioxane, d'élastomère d'organopolysiloxane enrobée de résine de silicone, une poudre une poudre de particules organosiliconées ;
- des poudres de polyamide, tel que de Nylon®, en particulier Nylon 12 ;

et leur(s) mélange(s).

De telles charges sphériques peuvent être enrobées avec un agent de traitement hydrophobe. L'agent de traitement hydrophobe peut être choisi parmi les acides gras comme l'acide stéarique ; les savons métalliques comme le dimyristate d'aluminium, le sel d'aluminium du glutamate de suif hydrogéné ; les acides aminés ; les acides aminés N-acylés ou leurs sels ; la lécithine, le trisostéaryle titanate d'isopropyle, et leurs mélanges. Les acides aminés N-acylés peuvent comprendre un groupe acyle ayant de 8 à 22 atomes de carbones, comme par exemple un groupe 2-éthyl hexanoyle, caproyle, lauroyle, myristoyle, palmitoyle, stéaroyle, cocoyle. Les sels de ces composés peuvent être les sels d'aluminium, de magnésium, de calcium, de zirconium, de zin, de sodium, de potassium. L'acide aminé peut être par exemple la lysine, l'acide glutamique, l'alanine. Le terme alkyl mentionné dans les composés cités précédemment désigne notamment un groupe alkyle ayant de 1 à 30 atomes de carbone, de préférence ayant de 5 à 16 atomes de carbone.

Plus particulièrement, la (les) charge(s) sphérique(s) est (sont) avantageusement choisie(s) parmi les poudres de (co)polymères acryliques, et leurs dérivés, en particulier une poudre de polyméthacrylate de méthyle, les poudres de polyuréthane, en particulier une poudre de copolymère de diisocyanate d'hexaméthylène et de triméthylol hexyl lactone, et leur(s) mélange(s).

### Poudres de silice

Comme poudre de silice, on peut citer :

- les microsphères de silice poreuses vendues sous la dénomination SILICA BEADS SB-700 par la société MYOSHI ; "SUNSPHERE® H51", "SUNSPHERE® H33" par la société ASAHI GLASS ;
- les microsphères de silice amorphe enrobées de polydiméthylsiloxane vendues sous la dénomination "SA SUNSPHERE® H 33", "SA SUNSPHERE® H53" par la société ASAHI GLASS.

### Poudres de (co)polymères acryliques, et leurs dérivés

Comme poudre de (co)polymères acryliques, et en particulier de (co)polymère acrylate, on peut citer :

- les poudres de polyméthacrylate de méthyle vendus sous la dénomination COVABEAD® LH85 par la société WACKHERR ;
- les poudres de poly méthacrylate de méthyle/diméthacrylate d'éthylène glycol vendues sous la dénomination DOW CORNING 5640 MICROSPONGE® SKIN OIL ADSORBER par la société DOW CORNING ; GANZPEARL® GMP-0820 par la société GANZ CHEMICAL ;
- les poudres de polyméthacrylate d'allyle/diméthacrylate d'éthylène glycol vendues sous la dénomination POLY-PORE® L200, POLY-PORE® E200 par la société AMCOL Health and Beauty Solutions Inc. ;
- les poudres de copolymère diméthacrylate d'éthylène glycol/méthacrylate de lauryle vendues sous la dénomination POLYTRAP® 6603 par la société DOW CORNING ;
- les poudre de copolymère acrylate / éthylhexyl acrylate réticulé vendues sous la dénomination TECHPOLYMER ACP-8C par la société SEKISUI PLASTICS ;
- les particules creuses de (co-) polymère d'acrylonitrile expansées vendues sous la dénomination EXPANCEL par la société Expancel;

et leur(s) mélange(s).

Les polyméthacrylates de méthyle se présentent généralement sous la forme de particules sphériques creuses ou pleines de couleur blanche dont la taille moyenne en nombre D50est généralement à l'échelle du micromètre, en particulier varie de 5 à 20 microns et généralement varie de 7 à 15 microns.

A titre représentatif et non limitatif des polyméthacrylates de méthyle convenant à l'invention, on peut notamment citer les particules de polyméthyméthacrylate commercialisées par la société WACKHERR sous la dénomination Covabead LH 85 et celles commercialisées par la société NIHON JUNYAKU sous la dénomination JURYMER MB1.

Les particules creuses de (co-)polymère d'acrylonitrile expansées sont ainsi issues d'au moins un polymère, ou copolymère, d'acrylonitrile. Elles sont réalisées en tout polymère, ou copolymère, d'acrylonitrile, expansé et non irritant pour la peau.

Ces particules sont avantageusement de forme sphérique. La masse volumique des particules est choisie dans la gamme allant de 15 kg/m$^3$ à 200 kg/m$^3$ et mieux de 30 kg/m$^3$ à 120 kg/m$^3$, et encore mieux de 40 kg/m$^3$ à 80 kg/m$^3$. Pour obtenir cette faible masse volumique, on utilise avantageusement des particules de polymères, ou copolymères, expansés, à base d'acrylonitrile et de préférence d'un monomère acrylique ou styrénique et/ou de chlorure de vinylidène.

On peut par exemple utiliser un copolymère contenant : de 0 % à 60 % de motifs dérivés du chlorure de vinylidène, de 20 % à 90 % de motifs dérivés d'acrylonitrile et de 0 % à 50 % de motifs dérivés d'un monomère acrylique ou styrénique, la somme des pourcentages (en poids) étant égale à 100. Le monomère acrylique est par exemple un acrylate ou méthacrylate de méthyle ou d'éthyle. Le monomère styrénique est par exemple l'-méthyl-styrène ou le styrène.

De préférence, les particules utilisées dans la présente invention sont des particules creuses d'un copolymère expansé de chlorure de vinylidène et d'acrylonitrile, un copolymère expansé de chlorure de vinylidène, d'acrylonitrile et de méthacrylate, et leur mélange. Ces particules peuvent être sèches ou hydratées.

Les particules de l'invention peuvent être obtenues, par exemple, selon les procédés des brevets et demandes de brevet EP-56219, EP-348372, EP-486080, EP-320473, EP-112807 et US-3615972.

La cavité interne des particules contient en principe un gaz qui peut être de l'air, de l'azote ou un hydrocarbure comme l'isobutane ou l'isopentane, de préférence l'isobutane.

De façon avantageuse, les particules de l'invention ont une granulométrie allant de 1 μm à 80 μm et encore mieux allant de 10 μm à 50 μm, et encore mieux de 20 μm à 40 μm.

Les particules utilisables dans l'invention sont par exemple les microsphères de terpolymère expansé de chlorure de vinylidène, d'acrylonitrile et de méthacrylate, vendues sous la marque EXPANCEL par la société Expancel sous les références 551 DE 40 (granulométrie d'environ 40 μm), 551 DE 20 (granulométrie d'environ 20 μm et masse volumique d'environ 65 kg/m$^3$), 551 DE 12 (granulométrie d'environ 12 μm), 551 DE 80 (granulométrie d'environ 80 μm), 461 DE 50 (granulométrie d'environ 50 μm). On peut aussi utiliser des microsphères formées du même terpolymère expansé ayant une granulométrie d'environ 18 μm et une masse volumique d'environ 70 kg/m$^3$, appelées ci-dessous EL 23, ou ayant une granulométrie d'environ 34 μm et une masse volumique d'environ 20 kg/m$^3$, appelées ci-dessous EL 43.

Les particules creuses de (co-)polymères acrylonitrile expansées sont de préférence choisies parmi un copolymère expansé de chlorure de vinylidène et d'acrylonitrile, un copolymère expansé de chlorure de vinylidène, d'acrylonitrile et de méthacrylate, et leur mélange.

### Poudres de polyuréthane

La poudre de polyuréthane est avantageusement une poudre de copolymère de diisocyanate d'hexaméthylène et de triméthylol hexyl lactone.

Avantageusement, la composition selon l'invention contient une poudre de polyuréthane qui n'est pas filmogène, c'est-à-dire qui ne forme pas un film continu lorsqu'elle est déposée sur un support tel que la peau.

Une telle poudre de polyuréthane est notamment vendue sous les dénominations « PLASTIC POWDER D-400 », « PLASTIC POWDER D-800 », et « PLASTIC POWDER T-75 » par la société TOSHIKI.

Comme autre poudre de polyuréthane, on peut utiliser celle vendue sous la dénomination « PLASTIC POWDER CS-400 » par la société TOSHIKI.

### Poudres de silicone

La (les) poudre(s) de silicone est (sont) avantageusement choisie(s) parmi une poudre de polyméthylsilsesquioxane, d'élastomère d'organopolysiloxane enrobée de résine de silicone, une poudre une poudre de particules organosiliconées

### Poudre de polyméthylsilsesquioxane

La composition selon l'invention comprend au moins une charge siliconée, et de façon préférée, cette charge siliconée est une poudre de polyméthylsilsesquioxane.

La présence d'une telle charge permet notamment d'améliorer la tenue, et en particulier la tenue de la couleur du dépôt sur la peau ou les lèvres réalisé avec la composition, sans perdre en brillance. La composition selon l'invention permet également l'obtention d'un dépôt peu ou non collant.

Comme poudre de polyméthylsilsesquioxane, on peut utiliser celle commercialisée sous la dénomination TOSPEARL par la société Momentive Performance Materials, et notamment sous la référence Tospearl 145 A.

### Poudre d'élastomère d'organopolysiloxane enrobée de résine de silicone

La composition selon l'invention comprend au moins une poudre d'élastomère d'organopolysiloxane enrobée de résine de silicone, notamment de résine silsesquioxane, comme décrit par exemple dans le brevet US 5 538 793 dont le contenu est incorporé à titre de référence.

De telles poudres d'élastomères sont vendues sous les dénominations « KSP-100 », « KSP-101 », « KSP-102 », « KSP-103 », « KSP-104 », « KSP-105 » par la société Shin Etsu, et ont pour nom INCI « vinyl dimethicone/methicone silsesquioxane Crosspolymer ».

De façon préférée, la poudre d'élastomère d'organopolysiloxane enrobée de résine de silicone est un composé de nom INCI « vinyl dimethicone/methicone silsesquioxane Crosspolymer ».

Comme poudre de silicone élastomère, on peut citer les poudres vendues sous les dénominations "Trefil® Powder E-505C", "Trefil® Powder E-506C" par la société DOW CORNING.

*Particules organosiliconées*

Selon un mode de réalisation particulier de l'invention, on utilise des portions de sphères creuses en forme de "bols". Celles-ci peuvent être obtenues comme décrit dans la demande JP-2003 128 788, des portions de sphères creuses en forme de fer à cheval sont aussi décrites dans la demande JP-A-2000-191789, ou encore dans la demande EP1579841.

Comme particules concaves de portions de sphères utilisables selon l'invention, on peut notamment citer :

- les particules constituées de l'organosilicone réticulé TAK-110 (polymère réticulé méthylsilanol/silicate) de la société TAKEMOTO OIL & FAT , en forme de bol , de largeur 2,5 $\mu$m, de hauteur 1,2 $\mu$m et d'épaisseur 150 nm (particules vendues sous la dénomination NLK-506 par la société Takemoto Oil & Fat) ;
- les particules constituées de l'organosilicone réticulé TAK-110 (polymère réticulé méthylsilanol/silicate) de la société TAKEMOTO OIL & FAT , en forme de bol , de largeur 2,5 $\mu$m, de hauteur 1,5 $\mu$m et d'épaisseur 350 nm ;
- les particules constituées de l'organosilicone réticulé TAK-110 (polymère réticulé méthylsilanol/silicate) de la société TAKEMOTO OIL & FAT , en forme de bol , de largeur 0,7 $\mu$m, de hauteur 0,35 $\mu$m et d'épaisseur 100 nm ;
- les particules constituées de l'organosilicone réticulé TAK-110 (polymère réticulé méthylsilanol/silicate) de la société TAKEMOTO OIL & FAT , en forme de bol , de largeur 7,5 $\mu$m, de hauteur 3,5 $\mu$m et d'épaisseur 200 nm.

*Poudres de Polyamide*

De préférence, les particules de polyamide présentent une taille moyenne en nombre allant de 50 nm et 350 microns, mieux entre 100 nm et 100 microns, et encore plus préférentiellement entre 0,5 et 100 microns.

Les particules de polyamide sont choisies parmi les particules de nylon 12.

Comme poudre de polyamide, on peut aussi citer les poudres de nylon vendues sous la dénomination ORGASOL® 2002 EXS NAT COS par la société ARKEMA.

*Charges lamellaires*

La phase pulvérulente peut comprendre au moins une charge lamellaire.

La (Les) charge(s) lamellaire(s) est (sont) de préférence minérales.

La (Les) charge(s) lamellaire(s) utilisable(s) dans les compositions selon l'invention est (sont) de préférence choisie(s) parmi le talc, le mica naturel ou synthétique, certaines silices, les argiles tels que les silicate de magnésium et d'aluminium, les poudre de perlite, le kaolin, la bentone, le carbonate de calcium et l'hydrogéno-carbonate de magnésium, l'hydroxyapatite, le nitrure de bore, la fluorphlogopite, une poudre N-Lauroyl Lysine, et leurs mélanges.

*Particules de Perlite(s)*

La perlite provient généralement de verre naturel d'origine volcanique, de couleur gris-clair ou noir brillant, résultant du refroidissement rapide de la lave et qui se présente sous forme de petites particules ressemblant à de la perle. Lorsque chauffée au-delà de 800°C, la perlite a la particularité de perdre l'eau qu'elle contient et de prendre une forme expansée poreuse (représentant de quatre à vingt fois son volume initial), l'autorisant à absorber de grandes quantités de liquide, en particulier d'huile et d'eau. Elle revêt alors une couleur blanche.

D'origine minérale, la perlite est directement extraite du sol puis finement broyée pour obtenir une poudre blanche très fine : la poudre de perlite, ou particules de perlite.

Les particules de perlite(s), sont ainsi des particules de matériaux minéraux amorphes, avantageusement expansés, issus d'au moins une roche volcanique.

Ces particules comportent au moins deux éléments choisis parmi le silicium, l'aluminium et le magnésium.

Plus particulièrement, ces matériaux minéraux sont obtenus par expansion thermique d'une roche volcanique ou « effusives » comprenant de 1 à 10% en poids d'eau et de préférence 1 à 5% en poids d'eau et moins de 10% en poids de roche cristalline par rapport au poids total de la composition de la roche et de préférence suivie d'un broyage. La température du procédé d'expansion peut varier de 700 à 1500°C et de préférence de 800 à 1100 °C. On peut notamment utiliser le procédé d'expansion décrit dans le brevet US 5,002,698.

Les roches volcaniques ou « effusives » sont généralement produites par le refroidissement rapide du liquide magmatique au contact de l'air ou de l'eau (phénomène de trempe donnant une roche hyaline). Les roches volcaniques utilisables selon la présente invention sont choisies parmi celles définies selon la classification de Streckeisen (1974). Parmi ces roches volcaniques, on peut citer notamment les trachytes, les latites, les andésites, les basaltes, les rhyolites, les dacites. Les rhyolites et les dacites conviennent particulièrement, et encore plus particulièrement les rhyolites.

Les particules de perlites utilisables selon l'invention sont de préférence des aluminosilicates d'origine volcanique. Ils ont avantageusement comme composition :

70,0-75,0% en poids de silice $SiO_2$
12,0-15,0% en poids d'oxyde d'aluminium oxyde $Al_2O_3$
3,0-5,0% d'oxyde de sodium $Na_2O$
3,0-5,0% d'oxyde de potassium $K_2O$
0,5-2% d'oxyde de fer $Fe_2O_3$
0,2-0,7% d'oxyde de magnésium MgO
0,5-1,5% d'oxyde de calcium CaO
0,05 - 0,15% d'oxyde de titane $TiO_2$

Dans la mise en oeuvre de la présente invention, la perlite subit une première étape de broyage de manière à former des particules de perlite, séchée, puis calibrée. Le produit obtenu dit Perlite Ore est de couleur grise et de taille de l'ordre de 100 $\mu$m. La Perlite Ore est ensuite expansée (1000°C/2 secondes) pour donner des particules plus ou moins blanches. Lorsque la température atteint 850-900 °C, l'eau emprisonnée dans la structure du matériau se vaporise et entraîne l'expansion du matériau par rapport à son volume d'origine. Les particules de perlite expansées conformes à l'invention peuvent être obtenues par le procédé d'expansion décrit dans le brevet US 5,002,698.

De préférence, les particules de perlite utilisées sont ensuite broyées dans une deuxième étape de broyage afin de réduire davantage la taille des particules de perlite mis en oeuvre ; elles sont dans ce cas dites Expanded Milled Perlite (EMP). Elles ont de préférence une taille de particule définie par un diamètre médian $D_{50}$ allant de 0,5 à 50 $\mu$m et de préférence de 1 à 40 $\mu$m.

De manière préférentielle, les particules de perlite présentent une forme plaquettaire ; et sont en conséquence usuellement appelées charges lamellaires, par opposition aux charges sphériques, de forme globulaire.

Les particules de perlite présentent avantageusement un coefficient d'expansion de 2 à 70.

Les particules de perlite(s) utilisées selon l'invention sont notamment disponibles dans le commerce auprès de la société WORLD MINERALS sous la dénomination commerciale Perlite P1430, Perlite P2550, Perlite P2040, ou OpTiMat™ 1430 OR ou 2550 OR.

A titre représentatif de telles charges mises en oeuvre de façon préférée dans la cadre de la présente invention, peuvent notamment être citées le talc, le mica, la fluorphlogopite, la perlite, les argiles tels que le silicate d'aluminium et de magnésium, une poudre N-Lauroyl Lysine, et leur(s) mélange(s).

La (Les) charge(s) lamellaire(s) est (sont) avantageusement présente(s) dans une composition conforme à la présente invention à une teneur totale supérieure ou égale à 5% en poids, par rapport au poids total de la composition, par exemple compris entre 8 et 30% en poids, par rapport au poids total de la composition, de préférence compris entre 8 et 25% en poids, mieux entre 10 et 20% en poids.

La (les) charge(s) sphérique(s) et la (les) charge(s) lamellaire(s) est (sont) présente(s) à une teneur totale supérieure ou égale à 5% en poids, par rapport au poids total de la composition, par exemple compris entre 8 et 30% en poids, par rapport au poids total de la composition, de préférence compris entre 8 et 25% en poids, mieux entre 10 et 20% en poids.

**Matières colorantes**

Les compositions conformes à l'invention comprennent de préférence au moins une matière colorante.

Cette (ou ces) matière(s) colorante(s) est (ou sont) de préférence choisie parmi les matières pulvérulentes, les colorants liposolubles, les colorants hydrosolubles, et leurs mélanges.

De préférence, les compositions selon l'invention comportent au moins une matière colorante pulvérulente. Les matières colorantes pulvérulentes peuvent être choisies parmi les pigments et les nacres, de préférence parmi les pigments.

Les pigments peuvent être blancs ou colorés, minéraux et/ou organiques, enrobés ou non. On peut citer, parmi les pigments minéraux, des oxydes métalliques, en particulier le dioxyde de titane, éventuellement traité en surface, les oxydes de zirconium, de zinc ou de cérium, ainsi que les oxydes de fer, de titane, ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium.

Les nacres peuvent être choisies parmi les pigments nacrés blancs tels que le mica recouvert de titane ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

Les colorants liposolubles sont par exemple le rouge Soudan, le D&C Red 17, le D&C Green 6, le β-carotène, l'huile de soja, le brun Soudan, le D&C Yellow 11, le D&C Violet 2, le D&C Orange 5, le jaune quinoléine, le rocou.

De préférence, les pigments contenus dans les compositions selon l'invention sont choisis parmi des oxydes métalliques.

Ces matières colorantes peuvent être présentes en une teneur allant de 0,01 à 30 % en poids par rapport au poids total de la composition, en particulier de 6 à 22% en poids par rapport au poids total de la composition.

De préférence, la (les) matières colorante(s) est (sont) choisi(s) parmi un ou plusieurs oxyde(s) métallique(s) présent(s) à une teneur supérieure ou égale à 2% en poids par rapport au poids total de la composition, avantageusement comprise inclusivement entre 6 et 22% en poids par rapport au poids total de la composition.

### *Cire(s)*

La (ou les) cire(s) est (sont) d'une manière générale un composé lipophile, solide à température ambiante (25° C), à changement d'état solide/liquide réversible, ayant un point de fusion supérieur ou égal 30°C pouvant aller jusqu'à 200°C et notamment jusqu'à 120°C.

Au sens de l'invention, la température de fusion correspond à la température du pic le plus endothermique observé en analyse thermique (DSC) telle que décrite dans la norme ISO 11357-3 ; 1999. Le point de fusion de la cire peut être mesuré à l'aide d'un calorimètre à balayage différentiel (DSC), par exemple le calorimètre vendu sous la dénomination "DSC Q2000" par la société TA Instruments.

De préférence, les cires présentent une enthalpie de fusion $\Delta Hf$ supérieure ou égale à 70 J/g.

De préférence, les cires comportent au moins une partie cristallisable, visible par observations aux rayons X.

Le protocole de mesure est le suivant :
Un échantillon de 5 mg de cire disposé dans un creuset est soumis à une première montée en température allant de -20 °C à 120 °C, à la vitesse de chauffe de 10 °C / minute, puis est refroidi de 120 °C à -20 °C à une vitesse de refroidissement de 10 °C / minute et enfin soumis à une deuxième montée en température allant de -20 °C à 120 °C à une vitesse de chauffe de 5 °C / minute. Pendant la deuxième montée en température, on mesure les paramètres suivants :

- le point de fusion ($T_f$) de la cire, tel que précédemment évoqué correspondant à la température du pic le plus endothermique de la courbe de fusion observé, représentant la variation de la différence de puissance absorbée en fonction de la température,
- $\Delta Hf$ : l'enthalpie de fusion de la cire correspondant à l'intégrale de l'ensemble de la courbe de fusion obtenue. Cette enthalpie de fusion de la cire est la quantité d'énergie nécessaire pour faire passer le composé de l'état solide à l'état liquide. Elle est exprimée en J/g.

La ou les cire(s) peut (peuvent) être hydrocarbonée(s), fluorée(s) et/ou siliconée(s) et être d'origine végétale, minérale, animale et/ou synthétique.

Lorsque présente(s), la ou les cire(s) est (sont) présente(s) à une teneur totale de préférence strictement inférieure à 10 % en poids, mieux à 5 % en poids, voire à 2 % en poids, par rapport au poids total de la composition.

### Cire dure

La composition peut comprendre au moins une cire dure.

Par « cire dure » au sens de la présente invention, on entend une cire ayant une température de fusion allant de 65 à 120°C, plus préférentiellement entre 70 et 100°C.

Avantageusement, par cire « dure » au sens de la présente invention, on entend une cire présentant à 20 °C, une dureté supérieure à 5 MPa, notamment allant de 5 à 30 MPa, de préférence supérieure à 6 MPa, mieux encore allant de 6 à 25 MPa.

Pour effectuer ces mesures de dureté, la cire est fondue à une température égale au point de fusion de la cire + 20 °C. On place pour cela 30g de cire dans un bêcher de 100ml de diamètre égal à 50mm, lui-même positionné sur une plaque d'agitation magnétique chauffante.

Une quantité d'environ 15g de cire fondue est coulée dans un récipient en inox de 80 mm de diamètre et de profondeur 15mm préalablement chauffé à 45°C dans une étuve. On laisse ensuite la cire recristalliser dans une pièce thermostatée à 20°C pendant 24 heures avant de faire la mesure.

Les propriétés mécaniques de la cire ou du mélange de cires sont déterminées dans une pièce thermostatée à 20°C à l'aide du texturomètre vendu sous la dénomination TA-XT2i par la société Swantech, équipé d'un cylindre en inox d'un diamètre de 2 mm.

La mesure comporte 3 étapes : une première étape après détection automatique de la surface de l'échantillon où le mobile se déplace à la vitesse de mesure de 0,1 mm/s, et pénètre dans la cire à une profondeur de pénétration de 0,3 mm, le logiciel note la valeur de la force maximale atteinte ; une seconde étape dite de relaxation ou le mobile reste à cette position pendant une seconde et où on note la force après 1 seconde de relaxation ; enfin une 3ème étape dite de retrait ou le mobile revient à sa position initiale à la vitesse de 1 mm/s et on note l'énergie de retrait de la sonde (force négative).

La valeur de la dureté correspond à la force de compression maximale mesurée en Newton divisée par la surface du cylindre du texturomètre exprimée en mm2 en contact avec la cire. La valeur de dureté obtenue est exprimée en méga-pascals ou MPa.

A titre d'exemples de cire dure, on peut notamment citer la cire de Carnauba, la cire de candelilla, la cire BIS-PEG-12 DIMETHICONE CANDELILLATE comme, par exemple, la Siliconyl Candellila Wax commercialisée par la société KOSTER KEUNEN, la cire de Jojoba hydrogénée telle que, par exemple, celle commercialisée par la société DESERT WHALE, l'huile de palme hydrogénée telle que celle commercialisée par la société SIO, la cire de son de riz, la cire de Sumac, les cires de cérésine, la cire de laurier, la cire d'insecte Chinois, la cire de Shellac, l'huile d'olive hydrogénée telle que la Waxolive de la société SOLIANCE, les cires obtenues par hydrogénation d'huile d'olive estérifiée avec des alcool gras à chaîne en C12 à C18 telle que celles vendues par la société SOPHIM sous les dénominations commerciales Phytowax Olive 12L44, 14L48, 16L55 et 18L57, les cires obtenues par hydrogénation d'huile de ricin estérifiée avec l'alcool cétylique ou béhénique comme par exemple celle qui sont vendues sous les dénominations Phytowax Ricin 16 L 64 et Phytowax Ricin 22 L 73 par la société SOPHIM, la cire de Cameline hydrogénée, la cire d'Ouricury, la cire de Montan, les cires d'ozokerite comme, par exemple, la Wax SP 1020 P commercialisée par la société Strahl & Pitsch, les cires microcristallines comme, par exemple, celle vendue sous la dénomination commerciale Microwax HW par la société PARAMELT, les triglycérides d'acides laurique, palmitique, cétylique et stéarique (nom INCI : coco-glycérides hydrogénés) tel que, par exemple, celui vendu sous la dénomination commerciale Softisan 100 par la société SASOL, les cires de polyméthylène comme, par exemple, celle vendue sous la dénomination commerciale Cirebelle 303 par la société SASOL, les cires de polyéthylène comme, par exemple, celles vendues sous les dénominations commerciales Performalene 400 polyethylene, Performalene 655 polyethylene et Performalene 500-L polyethylene par la société New Phase Technologies, les cires d'alcool-polyéthylène comme, par exemple, celle commercialisée sous le nom Performacol 425 Alcohol par la société BARECO, le copolymère éthylène/acide acrylique 95/5 vendu sous la dénomination commerciale cire AC 540 par la société Honeywell, l'hydroxystéarate d'hydroxyoctacosanyle comme, par exemple, celui vendu sous la dénomination commerciale l'Elfacos C 26 par la société AKZO, le stéarate d'octacosanyle comme, par exemple, celui commercialisé sous la dénomination Kester Wax K 82 H par la société KOSTER KEUNEN, le stéarate de stéaryle comme, par exemple, celui commercialisé sous la dénomination Liponate SS par la société LIPO CHEMICALS, le distéarate de pentaérythritol comme, par exemple, celui commercialisé sous la dénomination Cutina PES par la société COGNIS, le mélange d'adipate de dibéhényle, d'adipate de dioctadécyle et d'adipate de di-eicosanyle (nom INCI adipate de dialkyle en $C_{18-22}$), le mélange d'adipate de dilauryle et d'adipate de ditétradécyle (nom INCI : adipate de dialkyle en $C_{12-14}$), le mélange de sébaçate de dioctadécyle, de sébaçate didocosyle et de sébaçate dieicosyle (nom INCI : sébacate de dialkyle en $C_{18-22}$), le mélange d'octadécanedioate de dioctadécyle, d'octanedioate de didocosyle et d'octanedioate de dieicosyle (nom INCI : octanedioate de dialkyle en $C_{18-22}$) comme, par exemple, ceux commercialisés par la société COGNIS, le tétrastéarate de pentaérythrityle comme, par exemple, le Liponate PS-4 de la société Lipo Chemicals, le stéarate de tétracontanyle comme, par exemple, la Kester Wax K76 H de la société KOSTER KEUNEN, le benzoate de stéaryle comme, par exemple, le Finsolv 116 de la société FINETEX, le fumarate de béhényle comme, par exemple, le Marrix 222 de la société AKZO BERNEL, le tétrastéarate de di-(triméthylol-1,1,1-propane) comme, par exemple, celui qui est proposé sous la dénomination « HEST 2T-4S » par la société HETERENE, le distéarate de didotriacontanyle comme, par exemple, la Kester Wax K82D de la société KOSTER KEUNEN, le montanate de polyéthylène glycol à 4 motifs oxyéthylène (PEG-4) comme, par exemple, celui qui est vendu sous la dénomination commerciale Clariant Licowax KST1, le disalycilate d'hexanediol comme, par exemple, la Betawax RX-13750 commercialisée par la société CP Hall, l'hexastéarate de dipentaérythrytol comme, par exemple, celui qui est vendu sous la dénomination commerciale Hest 2P-6S par la société HETERENE, le tétrabéhénate de ditriméthylolpropane comme, par exemple, celui qui est vendu sous la dénomination commerciale Hest 2T-4B par la société HETERENE, les esters de Jojoba comme, par exemple, celui qui est vendu sous la dénomination commerciale Floraester HIP par la société FLORATECH, les mélanges d'acide carboxylique linéaire (C20-40)/ hydrocarbures saturés (nom INCI : C20-40 acid polyethylene) comme, par exemple, Performacid 350 acid de la société NEW PHASE TECHNOLOGIES, la cire synthétique de type Fischer-Tropsch telle que celle commercialisée sous la référence Rosswax 100 par la société ROSS,, l'alcool stéarique, l'alcool béhénique, le carbonate de dioctadécyle comme, par exemple, Cutina KE 3737, le polybéhénate de saccharose comme, par exemple, le Crodaderm B de la société CRODA, et leurs mélanges.

On peut également utiliser les cires citées ci-dessus sous la forme de mélanges disponibles dans le commerce, par exemple, sous les dénominations KOSTER KPC-56 (Mélange de 87,5 % en poids de stéarate de cétyle, de 7,5 % en poids d'alcool béhénique et de 5 % en poids de glycérides palm kernel), KPC-60 (Mélange de 87,5 % en poids de stéarate de stéaryle, de 7,5 % en poids d'alcool béhénique et de 5 % en poids de glycérides palm kernel), KPC-63 (Mélange de 87,5 % en poids de stéarate de béhényle, de 7,5 % en poids d'alcool béhénique et de 5 % en poids de glycérides palm kernel) et KPC-80 (Mélange de 86 % en poids de cire d'abeille synthétique, de 7,5 % d'huile végétale hydrogénée et de 6,5 % en poids d'alcool béhénique) de la société KOSTER KEUNEN.

On utilise de préférence des cires d'origine végétale telles que la cire de carnauba, la cire de candellila, la cire de jojoba hydrogénée, la cire de sumac, les cires obtenues par hydrogénation d'huile d'olive estérifiée avec les alcool gras à chaîne en C12 à C18 vendues par la société SOPHIM dans la gamme Phytowax (12L44, 14L48, 16L55 et 18L57), la cire de son de riz, les alcools stéarylique et béhéniques, la cire de laurier, la cire d'Ouricury.

La (les) cire(s) dure(s) est (sont) de préférence polaires.

Par cire « polaire », on entend une cire dont le paramètre de solubilité calculé au delà de son point de fusion $\delta_a$ est différent de 0 $(J/cm^3)^{1/2}$.

En particulier, par cire « polaire », on entend une cire dont la structure chimique est formée essentiellement, voire constituée, d'atomes de carbone et d'hydrogène, et comprenant au moins un hétéroatome fortement électronégatif tel qu'un atome d'oxygène, d'azote, de silicium ou de phosphore.

La définition et le calcul des paramètres de solubilité dans l'espace de solubilité tridimensionnel de HANSEN sont décrits dans l'article de C. M. HANSEN : "The three dimensionnal solubility parameters" J. Paint Technol. 39, 105 (1967).

Selon cet espace de Hansen :

- $\delta_D$ caractérise les forces de dispersion de LONDON issues de la formation de dipôles induits lors des chocs moléculaires ;
- $\delta_p$ caractérise les forces d'interactions de DEBYE entre dipôles permanents ainsi que les forces d'interactions de KEESOM entre dipôles induits et dipôles permanents;
- $\delta_h$ caractérise les forces d'interactions spécifiques (type liaisons hydrogène, acide/base, donneur/accepteur, etc.) ;
- $\delta_a$ est déterminé par l'équation :

$$\delta_a = \left(\delta_p{}^2 + \delta_h{}^2\right)^{1/2}$$

Les paramètres $\delta_p$, $\delta_h$, $\delta_D$ et $\delta_a$ sont exprimés en $(J/cm^3)^{1/2}$.

Lorsqu'une composition selon l'invention comprend au moins une cire dure, la teneur totale en cire(s) dure(s) est strictement inférieure à 10 % en poids, mieux à 5 % en poids, voire à 2% en poids par rapport au poids total de la composition.

Selon un mode de réalisation avantageux, la composition selon l'invention est exempte de cire(s) dure(s).

Une composition selon l'invention peut comprendre au moins une cire molle, c'est-à-dire dont la température de fusion est strictement inférieure à 50°C, et éventuellement dont la dureté est strictement inférieure à 5 MPa.

Toutefois, une composition selon l'invention comporte de préférence moins de 10 % en poids de cire(s) molle(s), de préférence moins de 5% en poids de cire(s) molle(s) voire moins de 2 % en poids de cire(s) molle(s), plus préférentiellement encore est exempte de cire(s) molle(s).

**Gélifiants**

Gélifiants hydrophiles

Les compositions selon la présente invention peuvent aussi contenir au moins un gélifiant hydrophile, ou hydrodoluble, ils peuvent être choisis parmi :

- les homo- ou copolymères d'acides acrylique ou méthacrylique ou leurs sels et leurs esters et en particulier les produits vendus sous les dénominations VERSICOL F® ou VERSICOL K® par la société ALLIED COLLOID, UTRAHOLD 8® par la société CIBA-GEIGY, les acides polyacryliques de type SYNTHALEN K,
- les copolymères d'acide acrylique et d'acrylamide vendus sous la forme de leur sel de sodium sous les dénominations RETEN® par la société HERCULES, les sels de sodium d'acides polyhydroxycarboxyliques vendus sous la dénomination HYDAGEN F® par la société HENKEL,
- les copolymères acide polyacryliques/acrylates d'alkyle de type PEMULEN,
- l'AMPS (Acide polyacrylamidométhyl propane sulfonique neutralisé partiellement à l'ammoniaque et hautement réticulé) commercialisé par la société CLARIANT,

- les copolymères AMPS/acrylamide de type SEPIGEL® ou SIMULGEL® commercialisés par la société SEPPIC, et
- les copolymères AMPS/méthacrylates d'alkyle polyoxyéthylénés (réticulés ou non), et leurs mélanges.
- les polymères associatifs et en particulier les polyuréthanes associatifs tels que le polymère $C_{16}$-OE$_{120}$-C$_{16}$ de la société ELEMENTIS (commercialisé sous le nom RHEOLATE FX1100, molécule à fonction uréthanne et poids moléculaire moyen en poids de 1300), OE étant un motif oxyéthyléné, le Rhéolate 205 à fonction urée vendu par la société RHEOX ou encore le Rhéolate 208 ou 204 (ces polymères étant vendus sous forme pure) ou le DW 1206B de chez RHOM & HAAS à chaîne alkyle en $C_{20}$ et à liaison uréthane, vendu à 20 % en matière active dans l'eau. On peut aussi utiliser des solutions ou dispersions de ces polyuréthanes associatifs notamment dans l'eau ou en milieu hydroalcoolique. A titre d'exemple, de tels polymères on peut citer, le RHEOLATE fx1010, le RHEOLATE FX1035 et le RHEOLATE 1070, le Rhéolate 255, le Rhéolate 278 et le Rhéolate 244 vendus par la société ELEMENTIS. On peut aussi utiliser le produit DW 1206F et le DW 1206J, ainsi que l'Acrysol RM 184 ou l'Acrysol 44 de la société RHOM & HAAS, ou bien encore le Borchigel LW 44 de la société BORCHERS,
- et leurs mélanges.

Certains polymères filmogènes hydrosolubles jouent également le rôle de gélifiant hydrosoluble.

Les gélifiants hydrophiles peuvent être présents dans les compositions selon l'invention en une teneur allant de 0,05 à 10% en poids par rapport au poids total de la composition, de préférence de 0,1 à 5% et mieux de 0,5 à 2 % en poids.

Une composition selon l'invention comprend avantageusement l'un des gélifiants susmentionnées, de préférence choisi parmi l'AMPS (Acide polyacrylamidométhyl propane sulfonique neutralisé partiellement à l'ammoniaque et hautement réticulé), les copolymères AMPS/acrylamide, et leur mélange.

Gélifiants lipophiles

Une composition selon l'invention peut comprendre au moins un gélifiant lipophile, ou liposoluble.

Le (les) gélifiant(s) utilisables peuvent être des gélifiants lipophiles organiques ou minéraux, polymériques ou moléculaires.

Comme gélifiants lipophiles minéraux, on peut citer les argiles, les argiles modifiées, telle que la Bentone 38 VCG par la société ELEMENTIS, et la silice pyrogénée éventuellement traitée hydrophobe en surface

Les gélifiants lipophiles organiques polymériques sont par exemple les organopolysiloxanes élastomériques partiellement ou totalement réticulés, de structure tridimensionnelle, comme ceux commercialisés sous les dénominations de KSG6®, KSG16® et de KSG18® par la société SHIN-ETSU, de Trefil E-505C® et Trefil E-506C® par la société DOW-CORNING, de Gransil SR-CYC®, SR DMF10®, SR-DC556®, SR 5CYC gel®, SR DMF 10 gel® et de SR DC 556 gel® par la société GRANT INDUSTRIES, de SF 1204® et de JK 113® par la société GENERAL ELECTRIC ; l'éthylcellulose comme celle vendue sous la dénomination Ethocel® par la société DOW CHEMICAL ; les polycondensats de type polyamide résultant de la condensation entre (α) au moins un acide choisi parmi les acides dicarboxyliques comprenant au moins 32 atomes de carbone tels que les acides gras dimères et (β) un alkylène diamine et en particulier l'éthylène diamine, dans lequel le polymère polyamide comprend au moins un groupe acide carboxylique terminal estérifié ou amidifié avec au moins un mono alcool ou une mono amine comprenant de 12 à 30 atomes de carbone linéaires et saturés, et en particulier, les copolymères d'éthylène diamine/dilinoléate de stéaryle tel que celui commercialisé sous la dénomination Uniclear 100 VG® par la société ARIZONA CHEMICAL ; les polyamides siliconés du type polyorganosiloxane tels que ceux décrits dans les documents US-A-5 874 069, US-A-5,919,441, US-A-6,051,216 et US-A-5,981,680 comme par exemple ceux commercialisés sous la référence Dow Corning 2-8179 et Dow Corning 2-8178 Gellant par la société DOW CORNING. Les copolymères séquencés de type "dibloc", "tribloc" ou "radial" du type polystyrène/polyisoprène, polystyrène/polybutadiène tels que ceux commercialisés sous la dénomination Luvitol HSB® par la société BASF, du type polystyrène/copoly(éthylène-propylène) tels que ceux commercialisés sous la dénomination de Kraton® par la société SHELL CHEMICAL CO ou encore du type polystyrène/copoly(éthylène-butylène), les mélanges de copolymères tribloc et radial (en étoile) dans l'isododécane tels que ceux commercialisé par la société PENRECO sous la dénomination Versagel® comme par exemple le mélange de copolymère tribloc butylène/éthylène/styrène et de copolymère étoile éthylène/propylène/styrène dans l'isododécane (Versagel M 5960).

Les compositions selon l'invention peuvent comprendre également en tant que gélifiant lipophile un élastomère de silicone non émulsionnant. On peut encore citer parmi les gélifiants lipophiles les organogélateurs.

Une composition selon l'invention est de préférence exempte de gélifiant lipophile.

Actifs cosmétiques

Les compositions conformes à l'invention peuvent également comprendre au moins un actif cosmétique.

Comme actifs cosmétiques pouvant être utilisés dans les compositions conformes à l'invention, on peut citer notamment les antioxydants, les conservateurs, les parfums, les neutralisants, les émollients, les agents de coalescence, les hydratants, les vitamines et les filtres en particulier solaires, et leurs mélanges.

Bien entendu, l'homme du métier veillera à choisir les éventuels additifs complémentaires et/ou leur quantité de telle manière que les propriétés avantageuses de la composition selon l'invention ne soient pas, ou substantiellement pas, altérées par l'adjonction envisagée.

De préférence, la composition selon l'invention est non rincée. Avantageusement, la composition est une composition de maquillage et en particulier un mascara.

## Huile ou solvant organique

Les compositions selon l'invention peuvent comprendre au moins une huile ou solvant organique.

Les compositions selon l'invention peuvent en particulier comporter au moins une huile choisie parmi au moins une huile non volatile, au moins une huile volatile, et leur mélange.

### Huile non volatile

Par « huile » on entend un corps gras liquide à température ambiante et pression atmosphérique.

Par « huile non volatile », on entend une huile restant sur la peau ou la fibre kératinique à température ambiante et pression. Plus précisément, une huile non volatile présente une vitesse d'évaporation strictement inférieure à 0.01 $mg/cm^2/min$.

Pour mesurer cette vitesse d'évaporation on introduit dans un cristallisoir, de diamètre 7 cm, placée sur une balance se trouvant dans une grande enceinte d'environ $0.3m^3$ régulée en température, à une température de 25°C, et en hygrométrie, à une humidité relative de 50%, 15g d'huile ou de mélange d'huile à tester. On laisse le liquide s'évaporer librement, sans l'agiter, en assurant une ventilation par un ventilateur (PAPST-MOTOREN, référence 8550 N, tournant à 2700 tours par minute) disposé en position verticale au-dessus du cristallisoir contenant ladite huile ou ledit mélange, les pales étant dirigées vers le cristallisoir et à une distance de 20cm par rapport au fond du cristallisoir. On mesure à intervalles réguliers la masse d'huile restant dans le cristallisoir. Les vitesses d'évaporation sont exprimées en mg d'huile évaporée par unité de surface ($cm^2$) et par unité de temps (minute).

Ladite au moins une huile non volatile peut être choisie parmi les huiles hydrocarbonées et les huiles siliconées, et leurs mélanges, de préférence parmi les huiles hydrocarbonées.

Les huiles non volatiles hydrocarbonées convenant à la présente invention peuvent en particulier être choisies parmi :

- les huiles hydrocarbonées d'origine végétale telles que les triglycérides constitués d'esters d'acides gras et de glycérol dont les acides gras peuvent avoir des longueurs de chaînes variées de C4 à C28, ces derniers pouvant être linéaires ou ramifiés, saturés ou insaturés ; ces huiles sont notamment les huiles de germe de blé, de tournesol, de pépins de raisin, de sésame, de maïs, d'abricot, de ricin, de karité, d'avocat, d'olive, de soja, l'huile d'amande douce, de colza, de coton, de noisette, de macadamia, de jojoba, de palme, de luzerne, de pavot, de potimarron, de sésame, de courge, de colza, de cassis, d'onagre, de millet, d'orge, de quinoa, de seigle, de carthame, de bancoulier, de passiflore, de rosier muscat ; ou encore les triglycérides des acides caprylique/caprique comme ceux vendus par la société Stéarineries Dubois ou ceux vendus sous les dénominations de Miglyol 810®, 812® et 818® par la société Sasol ;
- les éthers de synthèse ayant de 10 à 40 atomes de carbone ;
- les hydrocarbures linéaires ou ramifiés, d'origine minérale ou synthétique autres que les polymères selon l'invention, tels que la vaseline, les polybutènes, les polydécènes, le squalane, et leurs mélanges ;
- les esters de synthèse comme les huiles de formule R1COOR2 dans laquelle R1 représente le reste d'un acide gras linéaire ou ramifié comportant de 1 à 40 atomes de carbone et R2 représente une chaîne hydrocarbonée notamment ramifiée contenant de 1 à 40 atomes de carbone à condition que R1 + R2 soit $\geq$ 10, comme par exemple l'huile de Purcellin (octanoate de cétostéaryle), le myristate d'isopropyle, le palmitate d'isopropyle, le benzoate d'alcool en C12 à C15, le laurate d'hexyle, l'adipate de diisopropyle, l'isononanoate d'isononyle, le palmitate de 2-éthyl-hexyle, l'isostéarate d'isostéarate, des octanoates, les décanoates ou ricinoléates d'alcools ou de polyalcools comme le dioctanoate de propylène glycol ; les esters hydroxylés comme le lactate d'isostéaryle, le malate de di-isostéaryle ; et les esters du pentaérythritol
- les alcools gras liquides à température ambiante à chaîne carbonée ramifiée et/ou insaturée ayant de 12 à 26 atomes de carbone comme l'octyl dodécanol, l'alcool isostéarylique, l'alcool oléique, le 2-hexyldécanol, le 2-butyloctanol, le 2-undécylpentadécanol ;
- les acides gras supérieurs tels que l'acide oléique, l'acide linoléique, l'acide linolénique et leurs mélanges.

Les huiles non volatiles siliconées convenant à la présente invention peuvent en particulier être choisies parmi :

- les huiles de silicone non volatiles utilisables dans la composition conforme à l'invention peuvent être les polydiméthylsiloxanes (PDMS) non volatiles, les polydiméthylsiloxanes comportant des groupements alkyle ou alcoxy, pendant et/ou en bout de chaîne siliconée, groupements ayant chacun de 2 à 24 atomes de carbone, les silicones phénylées comme les phényl triméthicones, les phényl diméthicones, les phényl triméthylsiloxy diphénylsiloxanes, les diphényl diméthicones, les diphényl méthyldiphényl trisiloxanes, les 2-phényléthyl triméthylsiloxysilicates.

Une composition selon l'invention comporte éventuellement au moins une huile non volatile hydrocarbonée d'origine végétale, telles que les triglycérides constitués d'esters d'acides gras et de glycérol dont les acides gras peuvent avoir des longueurs de chaînes variées de C4 à C28, en particulier l'huile de palme et de jojoba hydrogénée. Une composition selon l'invention est de préférence exempte d'huile(s) non volatile(s) siliconée(s).

Une composition selon l'invention est de préférence exempte d'huile non volatile. Toutefois, la teneur totale en huile(s) non volatile(s) dans une composition conforme à l'invention peut aller de 0,01 à 10 % en poids, en particulier de 0,1 à 8 % en poids, et de préférence de 0,25 à 5 % en poids par rapport au poids total de la composition.

Selon un mode de réalisation préféré, une composition selon l'invention comprend moins de 5% en poids d'huile(s) non volatile(s) par rapport au poids total de la composition.

*Huile volatile*

La composition selon l'invention peut comprendre au moins une huile volatile.

Par "huile volatile", on entend une huile (ou milieu non aqueux) susceptible de s'évaporer au contact de la peau en moins d'une heure, à température ambiante et pression atmosphérique. L'huile volatile est une huile cosmétique volatile, liquide à température ambiante. Plus précisément, une huile volatile présente une vitesse d'évaporation comprise entre 0.01 et 200 mg/cm$^2$/min, bornes incluses.

Cette huile volatile peut être hydrocarbonée.

L'huile volatile hydrocarbonée peut être choisie parmi les huiles hydrocarbonées ayant de 7 à 16 atomes de carbone.

La composition selon l'invention peut contenir un ou plusieurs alcane(s) ramifié(s) volatil(s). Par « un ou plusieurs alcane(s) ramifié(s) volatil(s) », on entend indifféremment « une ou plusieurs huile alcane(s) ramifié(s) volatile(s) ».

Comme huile volatile hydrocarbonée ayant de 7 à 16 atomes de carbone, on peut citer notamment les alcanes ramifiés en C8-C16 comme les iso-alcanes (appelées aussi isoparaffines) en C8-C16, l'isododécane, l'isodécane, l'isohexadécane et par exemple les huiles vendues sous les noms commerciaux d'Isopars ou de Permetyls, les esters ramifiés en C8-C16 comme le néopentanoate d'iso-hexyle, et leurs mélanges. De préférence, l'huile volatile hydrocarbonée ayant de 8 à 16 atomes de carbone est choisie parmi l'isododécane, l'isodécane, l'isohexadécane et leurs mélanges, et est notamment l'isododécane.

La composition selon l'invention peut contenir un ou plusieurs alcane(s) linéaire(s) volatil(s). Par « un ou plusieurs alcane(s) linéaire(s) volatil(s) », on entend indifféremment « une ou plusieurs huile alcane(s) linéaire(s) volatile(s) ».

Un alcane linéaire volatil convenant à l'invention est liquide à température ambiante (environ 25 °C) et à la pression atmosphérique (760 mm Hg).

Par « alcane linéaire volatil » convenant à l'invention, on entend un alcane linéaire cosmétique, susceptible de s'évaporer au contact de la peau en moins d'une heure, à température ambiante (25°C) et pression atmosphérique (760 mm Hg, c'est-à-dire 101 325 Pa), liquide à température ambiante, ayant notamment une vitesse d'évaporation allant de 0,01 à 15 mg/cm$^2$/min, à température ambiante (25°C) et pression atmosphérique (760 mm Hg).

Les alcanes linéaires, de préférence d'origine végétale, comprenant de 7 à 15 atomes de carbone, en particulier de 9 à 14 atomes de carbone, et plus particulièrement de 11 à 13 atomes de carbone.

A titre d'exemple d'alcane linéaire convenant à l'invention, on peut mentionner les alcanes décrits dans les demandes de brevets de la société Cognis WO 2007/068371, ou WO2008/155059 (mélanges d'alcanes distincts et différant d'au moins un carbone). Ces alcanes sont obtenus à partir d'alcools gras, eux-mêmes obtenus à partir d'huile de coprah ou de palme.

A titre d'exemple d'alcane linéaire convenant à l'invention, on peut citer le n-heptane (C7), le n-octane (C8), le n-nonane (C9), le n-décane (C10), le n-undécane (C11), le n-dodécane (C12), le n-tridécane (C13), le n-tétradecane (C14), le -pentadécane (C15), et leurs mélanges, et en particulier le mélange de n-undécane (C11) et de n-tridécane (C13) décrit à l'exemple 1 de la demande WO2008/155059 de la Société Cognis. On peut également citer le n-dodécane (C12) et le n-tétradécane (C14) vendus par Sasol respectivement sous les références PARAFOL 12-97 et PARAFOL 14-97, ainsi que leurs mélanges.

On pourra utiliser l'alcane linéaire seul ou en mélange d'au moins deux alcanes distincts et différant entre eux d'un nombre de carbone d'au moins 1, et notamment un mélange d'au moins deux alcanes linéaires comportant de 10 à 14 atomes de carbone distincts et différant entre eux d'un nombre de carbone d'au moins 2, et en particulier un mélange d'alcanes linéaires volatiles C11/C13 ou un mélange d'alcanes linéaires C12/C14, en particulier un mélange n-undécane/n-tridécane (un tel mélange peut être obtenu selon l'exemple 1 ou l'exemple 2 du WO 2008/155059).

En variante ou de façon additionnelle, la composition réalisée peut comprendre au moins une huile ou solvant siliconée volatile, compatible avec une utilisation cosmétique.

Par huile siliconée, on entend une huile contenant au moins un atome de silicium, et notamment contenant des groupes Si-O. Selon un mode de réalisation, ladite composition comprend moins de 10% en poids d'huile(s) siliconée(s) non volatiles, par rapport au poids total de la composition, mieux moins de 5% en poids, voire est exempte d'huile siliconée.

Comme huile volatile siliconée, on peut citer les polysiloxanes cycliques, les polysiloxanes linéaires et leurs mélanges. Comme polysiloxanes volatiles linéaires, on peut citer l'hexamethyldisiloxane, l'octamethyltrisiloxane, le decamethyltetrasiloxane, le tetradecamethylhexasiloxane et l'hexadecamethylheptasiloxane. Comme polysiloxanes volatiles cycliques, on peut citer l'hexamethylcyclotrisiloxane, l'octamethylcylotetrasiloxane, le decamethylcyclopentasiloxane et le dodecamethylcyclohexasiloxane.

En variante ou de façon additionnelle, la composition réalisée peut comprendre au moins une huile volatile fluorée.

Par huile fluorée, on entend une huile contenant au moins un atome de fluor.

Comme huile volatile fluorée, on peut citer le nonafluorométhoxybutane ou le perfluorométhylcyclopentane, et leurs mélanges.

Une composition selon l'invention est de préférence exempte d'huile non volatile. Toutefois, au moins huile volatile peut être présente en une teneur totale allant de 0.1% à 10% en poids. En particulier, l'huile volatile peut être présente dans la composition dans une teneur allant de 0.5 à 5 % en poids par rapport au poids total de la composition.

Selon un mode de réalisation préféré, une composition selon l'invention comprend moins de 5% en poids d'huile(s) volatile(s) par rapport au poids total de la composition.

## ENSEMBLE

Un ensemble de revêtement des fibres kératiniques adapté à l'invention peut comprendre un applicateur configuré pour appliquer ladite composition cosmétique de revêtement des fibres kératiniques, et le cas échéant un dispositif de conditionnement adapté à recevoir ladite composition.

## Applicateur

L'applicateur peut comprendre des moyens permettant de lisser et/ou séparer les fibres kératiniques, telles que les cils ou les sourcils, notamment sous forme de dents, de poils ou autres reliefs.

L'applicateur est agencé pour appliquer la composition sur les cils ou les sourcils, et peut comporter par exemple une brosse ou un peigne.

L'applicateur peut encore être utilisé pour la finition du maquillage, sur une région des cils ou des sourcils maquillée ou chargée de la composition.

La brosse peut comporter une âme torsadée et des poils pris entre les spires de l'âme, ou être réalisée autrement encore.

Le peigne est par exemple réalisé d'une seule pièce par moulage de matière plastique.

Dans certains exemples de réalisation, l'élément d'application est monté à l'extrémité d'une tige, laquelle peut être flexible, ce qui peut contribuer à améliorer le confort à l'application.

## Dispositif de conditionnement

Le dispositif de conditionnement peut comprendre un récipient destiné à loger la composition de revêtement des fibres kératiniques. Cette composition peut alors être prélevée dans le récipient en immergeant l'applicateur dans celui-ci.

Cet applicateur peut être solidaire d'un élément de fermeture du récipient. Cet élément de fermeture peut former un organe de préhension de l'applicateur. Cet organe de préhension peut former un capot à monter de façon amovible sur ledit récipient par tous moyens appropriés tels que par vissage, encliquetage, emmanchement ou autre. Un tel récipient peut donc loger de façon réversible ledit applicateur.

Ce récipient peut être éventuellement équipé d'un essoreur adapté à débarrasser un surplus de produit prélevé par l'applicateur.

Un procédé d'application de la composition selon l'invention sur les cils ou les sourcils peut également comporter les étapes suivantes :

- former un dépôt de la composition cosmétique sur les cils ou les sourcils,
- laisser le dépôt sur les cils ou les sourcils, le dépôt pouvant sécher.

Il est à noter que selon un autre mode de réalisation l'applicateur peut former un récipient de produit. Dans un tel cas un récipient peut par exemple être prévu dans l'organe de préhension et un canal interne peut relier intérieurement cet organe de préhension aux éléments en reliefs d'application.

Enfin il est à noter que l'ensemble de conditionnement et d'application peut se présenter sous la forme d'un kit, l'applicateur et le dispositif de conditionnement pouvant être logés séparément sous un même article de conditionnement.

Les exemples précédents et suivants sont donnés à titre illustratif de la présente invention, et ne sauraient en limiter la portée.

**EXEMPLES**

Une composition de mascara conforme à l'invention est décrite ci-dessous et comparée à une composition hors invention :

| Ingrédients avec teneurs en pourcentage | Composition A selon l'invention | Composition B comparative hors invention |
|---|---|---|
| STEARETH 2 (Brij 72 d'Uniqema) | 10 | - |
| STEARETH 20 (Brij 78P d'Uniqema) | 10 | - |
| Cire de CARNAUBA | - | 16 |
| Peg 30 Glyceryl stearate (TAGAT S de EVONIK) | - | 4 |
| COPOLYMERES ACRYLIQUE ET STYRÈNE/ACRYLIQUE EN EMULSION AQUEUSE A 40% DANS MELANGE EAU/BUTYLENE GLYCOL/SODIUM LAURYL ETHER SULFATE PROTEGE (SYNTRAN 5760 CG de INTERPOLYMER) | 25 | 25 |
| Pigments (oxydes de fer) | 10 | 10 |
| Eau | Qsp 100 | Qsp 100 |
| Conservateurs | Qs | Qs |

Ces compositions ont été préparées comme suit :
On pèse les ingrédients, on fait fondre à 80°C les Steareth 2 et 20 conformes à l'invention, on ajoute alors l'eau préalablement chauffé dans une bouilloire électrique à 95°C et les pigments. On mélange 5 minutes à 95°C sous Moritz.

On verse les conservateurs dans le mélange lorsque la température du mélange est inférieure ou égale à 45°C.

Le mascara ainsi obtenu est transféré dans un bocal clos pour éviter son séchage au contact de l'air, il faut alors attendre 24h pour vérifier l'homogénéité de la formule et la bonne dispersion des pigments.

1/ Vérification de la présence d'une phase lamellaire Lβ

La présence d'une phase lamellaire Lβ a tout d'abord été suspectée au moyen d'un microscope optique avec lumière polarisée croisée avec un agrandissement de 10.

Il est à noter que la caractérisation d'une phase lamellaire Lβ par la technique de diffraction aux grands angles précédemment expliquée devrait se faire essentiellement sur une combinaison système tensioactif et phase aqueuse afin d'éviter toute diffusion qui pourrait masquer les raies de phase lamellaire, susceptible de perturber les mesures. Ainsi, cette mesure devrait se faire sur une composition exempte de pigments et de charges, et préférentiellement sans dispersion de particules de polymère(s) filmogène(s). Cette mesure a été faite sur la composition suivante :

| Ingrédients avec teneurs en pourcentage | Composition A' selon l'invention |
|---|---|
| STEARETH 2 (Brij 72 d'Uniqema) | 7 |
| STEARETH 20 (Brij 78P d'Uniqema) | 15 |
| Eau | Qsp 100 |
| Conservateurs | Qs |

Le spectre de diffraction RX de cette composition selon l'invention A' montre trois raies fines à q égal 0.052, q égal 0.107, et q égal 0.16 Angstrom$^{-1}$ ce qui nous donne une période de la phase lamellaire de 120 Angstrom.

Il est à noter que bien que les teneurs en tensioactifs ont été quelques peu modifiées par rapport à l'exemple de composition selon l'invention A donné précédemment, un spectre de diffraction RX similaire, voire identique, serait obtenu avec les teneurs en tensioactifs de ladite composition A exempte de pigments.

2/ Protocoles et Résultats

La composition préparée est observée à l'œil nu et au microscope, puis testée sur une éprouvette de cils vierges, par application de ces compositions à l'aide d'une brosse.

La composition A conforme à l'invention est agréable à l'application, elle présente une texture fluide (viscosité à 25°C de 4.5 Pa.s mesurée par l'appareil Rhéomat RM100®) la composition gaine bien les cils, le maquillage est régulier, la frange de cils bien déployée. Elle présente en outre un noir de bonne intensité.

En outre, cette composition est stable à 4 et 45°C pendant deux mois.

Le composition B est elle trop liquide pour permettre une mesure rhéologique.

L'application de la composition A sur les cils est plus agréable que la composition B. Le playtime de la composition A est plus important que celui de la composition B, étant supérieur à 20 coups de brosses sans frein à l'application, ni formation de grains pour la composition B. De plus, le volume conféré par la composition A est plus important que celui conféré par la composition B.

**Autres exemples de système émulsionnant selon l'invention**

D'autres compositions selon l'invention utilisant des variantes de systèmes de tensioactifs conformes à l'invention ont été préparées et la présence de phase lamellaire vérifiée au d'un microscope optique à lumière polarisée croisée avec un agrandissement de 10.

Composition C (selon l'invention) :

| Ingrédients avec teneurs en pourcentage | Composition C selon l'invention |
|---|---|
| Sucrose tristéarate (RYOTO SUGAR ESTER S 370 de MITSUBISHI-KAGAKU FOODS) | 10 |
| Polysorbate 60 (Tween 60-SS-(TH) de Croda) | 10 |
| Eau | Qsp 100 |

Composition D (selon l'invention) :

| Ingrédients avec teneurs en pourcentage | Composition D selon l'invention |
|---|---|
| STEARETH 2 (Brij S2-SO-(TH) de Croda) | 10 |
| PEG-40 stéarate (MYRJ S40-FL-(TH) de Croda) | 10 |
| Eau | Qsp 100 |

Composition E (selon l'invention) :

| Ingrédients avec teneurs en pourcentage | Composition E selon l'invention |
|---|---|
| Stéarate de glycéryle (TEGIN 90 PELLETS de EVONIK GOLDSCHMIDT) | 10 |

(suite)

| Ingrédients avec teneurs en pourcentage | Composition E selon l'invention |
|---|---|
| PEG-40 stéarate (MYRJ S40-FL-(TH) de CRODA) | 10 |
| Eau | Qsp 100 |

Composition F (selon l'invention) :

| Ingrédients avec teneurs en pourcentage | Composition F selon l'invention |
|---|---|
| STEARETH 2 (Brij S2-SO-(TH) de CRODA) | 10 |
| PEG-200 GLYCERYL STEARATE (SIMULSOL 220 TM de Seppic) | 10 |
| Eau | Qsp 100 |

RESULTATS

Pour les compositions 2C à 2F selon l'invention, on a observé pour chacune la formation d'une phase lamellaire Lβ (présence de croix de malte, structures biréfringentes et stries huileuses).

**Exemple de systèmes tensioactifs hors invention**

Composition G hors invention :

| Ingrédients avec teneurs en pourcentage | Composition G comparative hors invention |
|---|---|
| STEARETH 2 (Brij S2-SO-(TH) de CRODA) | 10 |
| PHOSPHATE DE CETYLE (AMPHISOL K de DSM Nutritional Products) | 10 |
| Eau | Qsp 100 |

Un tel système tensioactif associant un stéareth 2 à un tensioactif non conforme à l'invention ne conduit pas à la formation d'une phase lamellaire Lβ, contrairement aux compositions selon l'invention.

Composition H hors l'invention :

| Ingrédients avec teneurs en pourcentage | Composition H comparative hors invention |
|---|---|
| STEARETH 2 (BRIJ S2-SO-(TH) de CRODA) | 10 |
| STEARETH 20 (BRIJ S20-PA-(SG) de CRODA) | 3 |
| Eau | Qsp 100 |

La présence de seulement 13% en poids d'un mélange de tensioactifs de HLB à 25 °C inférieure à 8 et de HLB à 25 °C supérieure ou égale à 8, strictement inférieure à la teneur de 15% en poids conforme à l'invention, ne permet pas de former une phase lamellaire Lβ, contrairement aux compositions selon l'invention.

Il est entendu que dans le cadre de la présente invention les pourcentages pondéraux donnés pour un composé ou une famille de composés, sont toujours exprimés en poids de matière sèche du composé en question.

Dans toute la demande, le libellé « comprenant un » ou « comportant un » signifie « comprenant au moins un » ou « comportant au moins » un sauf si le contraire est spécifié.

**Revendications**

**1.** Composition cosmétique de revêtement des fibres kératiniques, comportant :

- une phase aqueuse,
- un système tensioactif présent à une teneur totale supérieure ou égale à 15% en poids par rapport au poids total de la composition, ledit système tensioactif comprenant :

  i) au moins un tensioactif non ionique de valeur HLB à 25°C inférieure à 8, et
  ii) au moins un tensioactif non ionique de valeur HLB à 25°C supérieure ou égale à 8, formant ensemble une phase lamellaire Lβ,

  le (les) tensioactif(s) non ionique(s) de valeur HLB à 25°C supérieure ou égale à 8 est (sont) présent(s) à une teneur supérieure ou égale à 5% en poids par rapport au poids total de la composition, de préférence compris entre 7 et 30%, de préférence compris entre 10 et 20% en poids par rapport au poids total de la composition
  le (les) tensioactif(s) non ionique(s) de valeur HLB à 25°C inférieure à 8 est (sont) présent(s) à une teneur supérieure ou égale à 5% en poids par rapport au poids total de la composition, de préférence compris entre 7 et 30%, de préférence compris entre 10 et 20% en poids par rapport au poids total de la composition
  le (les) tensioactif(s) non ionique(s) de valeur HLB à 25°C inférieure à 8 et le (les) tensioactif(s) non ionique(s) de valeur HLB à 25°C supérieure ou égale à 8 sont présents à une teneur totale supérieure ou égale à 15%, en particulier compris entre 16 et 40 %, de préférence entre 18 et 30% en poids, par rapport au poids total de la composition
- au moins une dispersion aqueuse de particules d'au moins un polymère filmogène, le (les) polymère(s) filmogène(s) étant de préférence présent(s) à une teneur en matière sèche supérieure ou égale à 5% en poids, de préférence à 10 % en poids, par rapport au poids total de ladite composition.
- étant entendu que ladite composition peut comprendre au moins une cire, la (les) cire(s), lorsque présente(s), étant toutefois présente(s) à une teneur totale strictement inférieure à 10% en poids par rapport au poids total de la composition.

2.  Composition selon la revendication 1, dans laquelle moins un parmi le (les) tensioactif(s) non ionique(s) de valeur HLB à 25°C inférieure à 8, et/ou, de préférence et, au moins un parmi le (les) tensioactif(s) de valeur HLB à 25°C supérieure ou égale à 8 répond à la formule suivante (I) :

$$(ALK\text{-}[C(O)]_a\text{-}[O]_b)_c\text{-}X \qquad (I)$$

Formule (I) dans laquelle :

- ALK est un groupe alkyle en $C_7$-$C_{23}$, de préférence en $C_{11}$-$C_{21}$, plus préférentiellement en $C_{15}$-$C_{17}$,
- a et b des nombres entiers compris entre 0 et 100, c est un nombre entier compris entre 1 et 100, en particulier compris entre 1 et 3, de préférence égal à 1, a et b étant de préférence égal à 0,
- X est un groupe (poly)oxyalkylène éventuellement substitué et/ou terminé par un groupe hydroxy, X étant de préférence un groupe oxyéthylène $(CH_2CH_2O)_n$ ou $(OCH_2CH_2)_n$ dans lequel n est supérieure ou égale à 1, par exemple compris entre 1 et 200, de préférence ledit groupe (poly)oxyalkylène étant un polyéthylène glycol ou étant le résultat d'au moins une substitution d'un groupe hydroxy, de préférence choisi parmi les (poly)glycérols.

3.  Composition selon la revendication 2, dans laquelle le groupe X est choisi parmi :

  i)

$$HO\text{-}(ALK\text{-}O)_z\text{-}CH2\text{-}CH[(OALK)_y\text{-}OH]\text{-}CH2\text{-}(O\text{-}ALK)_x\text{-}(*)$$

  dans laquelle :

  - ALK identique ou différent représentant un groupe alkylène en C1-C6, en particulier en C1-C4, de préférence l'éthylène,
  - x, y, z étant un entier compris entre 0 et 200, étant entendu que x+y+z différent de 0, de préférence x+y+z étant inclusivement compris entre 1 et 150, en particulier entre 20 et 60 ;

  ii)

$$H\text{-}(ALK\text{-}O)_x\text{-}(*) \text{ et } H\text{-}(O\text{-}ALK)_x\text{-}(*),$$

de préférence est H-(O-ALK)$_x$-(*) dans laquelle :

- ALK identique ou différent représentant un groupe éthylène en C1-C6, en particulier en C1-C4, de preference l'éthylène,
- x est un entier différent de 0 et de préférence compris entre 1 et 200.

4. Composition selon l'une quelconque des revendications 1 à 3, dans laquelle le (les) tensioactif(s) non ionique(s) de valeur HLB à 25°C inférieure à 8, et/ou, de préférence et, le (les) tensioactif(s) non ionique(s) de valeur HLB à 25°C supérieure ou égale à 8, répondent à la formule (I') suivante :

$$ALK\text{-}(O\text{-}CH_2\text{-}CH_2)_n\text{-}OH \text{ (I')}$$

Formule (I') dans laquelle :

- ALK est un groupe alkyle en C$_8$-C$_{24}$, de préférence en C$_{12}$-C$_{22}$, plus préférentiellement en C$_{16}$-C$_{18}$,

n étant un entier différent de 0, compris entre 1 et 200, de préférence compris entre 1 et 10, mieux entre 2 et 6 pour le (les) tensioactif(s) non ionique(s) de valeur HLB à 25°C inférieure à 8, et de préférence strictement supérieur à 10, en particulier compris entre 15 et 200, pour le (les) tensioactif(s) non ionique(s) de valeur HLB à 25°C supérieure ou égale à 8.

5. Composition selon l'une quelconque des revendications 1 à 4, dans laquelle le (les) tensioactif(s) non ionique(s) de valeur HLB à 25°C inférieure à 8 est (sont) choisi(s) parmi :

- les esters et éthers d'oses éventuellement (poly)oxyalkylénés, de préférence (poly)oxyalkylénés ;
- les esters d'acides gras, notamment en C$_8$-C$_{24}$, et de préférence en C$_{16}$-C$_{22}$, et de polyol éventuellement (poly)oxyalkyléné, de préférence (poly)oxyalkyléné, notamment de glycérol (poly)oxyalkyléné ou de sorbitol oxyalkyléné, de préférence de glycérol (poly)oxyalkyléné ;
- les alcools éventuellement (poly)oxyalkylénés, de préférence (poly)oxyalkylénés ;

et leurs mélanges ; de préférence parmi les alcools éventuellement (poly)oxyalkylénés, de préférence (poly)oxyalkylénés comportant de préférence de 1 à 10 motifs oxyéthylènes.

6. Composition selon l'une quelconque des revendications 1 à 5, dans laquelle le (les) tensioactif(s) non ionique(s) de valeur HLB à 25°C inférieure à 8 comprend un alcool éventuellement (poly)oxyalkyléné, de préférence (poly)oxyalkyléné comprenant un éther d'alcool gras en C$_8$-C$_{24}$ et de polyéthylène glycol, ledit éther comprenant entre 1 à 10, mieux entre 2 et 6, motifs d'éthylène glycol.

7. Composition selon l'une quelconque des revendications 1 à 6, dans laquelle le (les) tensioactif(s) non ionique(s) de valeur HLB à 25°C supérieure ou égale à 8, de préférence supérieure ou égale à 10, est (sont) choisi(s) parmi :

- les éthers de glycérol (poly)oxyalkylénés,
- les alcools (poly)oxyalkylénés,
- les esters d'acide gras et de polyéthylène glycol (poly)oxyalkylénés,
- les esters d'acide gras et d'éthers de glycérol (poly)oxyalkylénés,
- les esters d'acide gras et d'éthers de sorbitol (poly)oxyalkylénés,

et leur(s) mélange(s) ; de préférence parmi les alcools (poly)oxyalkylénés comportant de préférence plus de 10 motifs oxyéthylènes, en particulier entre 15 et 200 motifs oxyéthylènes.

8. Composition selon l'une quelconque des revendications 1 à 7, dans laquelle le (les) tensioactif(s) non ionique(s) de valeur HLB à 25°C supérieure ou égale à 8 comprend un alcool (poly)oxyalkyléné comprenant au moins un éther d'alcool gras en C$_8$-C$_{24}$ et de polyéthylène glycol, ledit éther comprenant plus de 10 motifs d'éthylène glycol, mieux entre 15 et 200 motifs d'éthylène glycol.

9. Composition selon l'une quelconque des revendications précédentes, dans laquelle la phase aqueuse représente de 30 à 70% en poids, de préférence de 40 à 60 % en poids, par rapport au poids total de la composition.

**10.** Composition selon l'une quelconque des revendications 2 à 9, dans laquelle le (les) tensioactif(s) non ionique(s) de valeur HLB à 25°C inférieure à 8 de formule (I) et le (les) tensioactif(s) non ionique(s) de valeur HLB à 25°C supérieure ou égale à 8 de formule (I) sont présents à une teneur totale supérieure ou égale à 15%, en particulier compris entre 16 et 40%, de préférence entre 18 et 30% en poids, par rapport au poids total de la composition.

**11.** Composition selon l'une quelconque des revendications précédentes, dans laquelle le (les) tensioactif(s) non ionique(s) de valeur HLB à 25°C inférieure à 8, de préférence de formule (I), et le (les) tensioactif(s) non ionique(s) de valeur HLB à 25°C supérieure ou égale à 8, de préférence de formule (I), sont présents à une teneur totale respective telle que le rapport pondérale du (des) tensioactif(s) non ionique(s) de valeur HLB à 25°C inférieure à 8, de préférence de formule (I), sur le (les) tensioactif(s) non ionique(s) de valeur HLB à 25°C supérieure ou égale à 8, de préférence de formule (I), va de 1/5 à 5, de préférence de 1/3 à 3, de préférence de 2/3 à 3/2.

**12.** Composition selon l'une quelconque des revendications précédentes, dans laquelle les particules de polymère(s) filmogène(s) présente(s) sous forme de dispersion(s) aqueuse(s) est (sont) choisi(s) parmi les polymères synthétiques, de type radicalaire ou de type polycondensat, les polymères d'origine naturelle, et leurs mélanges.

**13.** Composition selon l'une quelconque des revendications précédentes, dans laquelle les particules de polymère(s) filmogène(s) présente(s) sous forme de dispersion(s) aqueuse(s) est (sont) choisi(s) parmi les dispersions de polymères acryliques, les dispersions de polyuréthanne, les dispersions de sulfopolyesters, les dispersions vinyliques, les dispersions de polyvinyl acétate, les dispersions de terpolymère vinyl pyrrolidone, diméthylaminopropyl méthacrylamide et chlorure de lauryldiméthylpropylméthacrylamidoammonium, les dispersions de polymère hybrides polyuréthane/polyacryliques, les dispersions de particules de type cœur-écorce et leurs mélanges, de préférence parmi les dispersions de polymères acryliques, dispersions de polymère hybrides polyuréthane/polyacryliques, et leurs dérivés, et leur(s) mélange(s), préférentiellement parmi les dispersions de polymères acryliques, en particulier styrène-acrylique, et les dispersions de polyuréthanne, en particulier polyester-polyuréthanne, et leurs dérivés, et leur(s) mélange(s).

**14.** Composition selon l'une quelconque des revendications précédentes, dans laquelle la teneur totale en particules de polymère(s) filmogène(s) et la teneur totale en système tensioactif sont telles que le rapport pondéral des particules de polymère(s) filmogène(s) / le système tensioactif est inférieur ou égal à 1.75, de préférence inférieur ou égal à 1.5, en particulier compris entre 1/2 et 1.25.

**15.** Composition selon l'une quelconque des revendications précédentes, comprenant au moins une matière colorante choisie parmi une ou plusieurs matière(s) pulvérulente(s), de préférence des oxydes métalliques, et en particulier des oxydes de fer.

**16.** Composition selon l'une quelconque des revendications précédentes, comprenant une viscosité à 25°C allant de 5 à 50 Pa.s.

**17.** Procédé de revêtement des fibres kératiniques, en particulier de maquillage des cils, comprenant une étape d'application d'une composition cosmétique de revêtement des fibres kératiniques selon l'une quelconque des revendications précédentes.

**Patentansprüche**

**1.** Kosmetische Zusammensetzung zur Beschichtung von Keratinfasern, umfassend:

- eine wässrige Phase,
- ein Tensidsystem, das in einem Gesamtgehalt größer oder gleich 15 Gew.-%, bezogen auf das Gesamtgewicht der Zusammensetzung, vorliegt, wobei das Tensidsystem Folgendes umfasst:

i) mindestens ein nichtionisches Tensid mit einem HLB-Wert bei 25 °C von weniger als 8 und
ii) mindestens ein nichtionisches Tensid mit einem HLB-Wert bei 25 °C größer oder gleich 8, die zusammen eine lamellare Phase Lβ bilden,

das nichtionische Tensid bzw. die nichtionischen Tenside mit einem HLB-Wert bei 25 °C größer oder gleich 8 in einem Gehalt größer oder gleich 5 Gew.-%, bezogen auf das Gesamtgewicht der Zusammensetzung, insbesondere zwischen 7 und 30 Gew.-%, vorzugsweise im Bereich von 10 bis 20 Gew.-%, bezogen auf das Gesamtgewicht der Zusammensetzung, vorliegt bzw. vorliegen,

das nichtionische Tensid bzw. die nichtionischen Tenside mit einem HLB-Wert bei 25 °C von weniger als 8 in einem Gehalt größer oder gleich 5 Gew.-%, bezogen auf das Gesamtgewicht der Zusammensetzung, insbesondere zwischen 7 und 30 Gew.-%, vorzugsweise im Bereich von 10 bis 20 Gew.-%, bezogen auf das Gesamtgewicht der Zusammensetzung, vorliegt bzw. vorliegen,

das nichtionische Tensid bzw. die nichtionischen Tenside mit einem HLB-Wert bei 25 °C von weniger als 8 und das nichtionische Tensid bzw. die nichtionischen Tenside mit einem HLB-Wert bei 25 °C größer oder gleich 8 in einem Gesamtgehalt größer oder gleich 15 Gew.-%, bezogen auf das Gesamtgewicht der Zusammensetzung, insbesondere zwischen 16 und 40 Gew.-%, noch besser zwischen 18 und 30 Gew.-%, bezogen auf das Gesamtgewicht der Zusammensetzung, vorliegen,

- mindestens eine wässrige Dispersion von Teilchen von mindestens einem filmbildenden Polymer, wobei das filmbildende Polymer bzw. die filmbildenden Polymere vorzugsweise in einem Feststoffgehalt größer oder gleich 5 Gew.-%, vorzugsweise 10 Gew.-%, bezogen auf das Gesamtgewicht der Zusammensetzung, vorliegt bzw. vorliegen,

- wobei es sich versteht, dass die Zusammensetzung mindestens ein Wachs enthalten kann, wobei das Wachs bzw. die Wachse, sofern vorhanden, jedoch in einem Gesamtgehalt, der streng weniger als 10 Gew.-%, bezogen auf das Gesamtgewicht der Zusammensetzung, beträgt, vorliegt bzw. vorliegen.

2. Zusammensetzung nach Anspruch 1, wobei das nichtionische Tensid bzw. die nichtionischen Tenside mit einem HLB-Wert bei 25 °C von weniger als 8 und/oder vorzugsweise mindestens eines des nichtionischen Tensids bzw. der nichtionischen Tenside mit einem HLB-Wert bei 25 °C größer oder gleich 8 der nachstehenden Formel (I) entspricht bzw. entsprechen:

$$(ALK-[C(O)]_a-[O]_b)_c-X \qquad (I)$$

wobei in der Formel (I):

- ALK für eine $C_7$-$C_{23}$-, vorzugsweise $C_{11}$-$C_{21}$- und weiter bevorzugt $C_{15}$-$C_{17}$-Alkylgruppe steht,
- a und b für ganze Zahlen zwischen 0 und 100 stehen, c für eine ganze Zahl zwischen 1 und 100, insbesondere zwischen 1 und 3, vorzugsweise gleich 1, steht, wobei a und b vorzugsweise gleich 0 sind,
- X für eine (Poly)oxyalkylengruppe, die gegebenenfalls durch eine Hydroxylgruppe substituiert und/oder terminiert ist, steht, wobei X vorzugsweise für eine Oxyethylengruppe $(CH_2CH_2O)_n$ oder $(OCH_2CH_2)_n$ steht, wobei n größer oder gleich 1 ist, beispielsweise zwischen 1 und 200 liegt, wobei es sich bei der (Poly)oxyalkylengruppe vorzugsweise um ein Polyethylenglykol oder das Ergebnis mindestens einer Substitution einer Hydroxylgruppe, vorzugsweise ausgewählt aus (Poly)glycerinen, handelt.

3. Zusammensetzung nach Anspruch 2, wobei die Gruppe X ausgewählt ist aus:

i)

$$HO-(ALK-O)_z-CH2-CH[(OALK)_y-OH]-CH2-(O-ALK)_x-(*)$$

wobei:

- ALK, das gleich oder verschieden sein kann, für eine C1-C6- und insbesondere C1-C4-Alkylengruppe, vorzugsweise Ethylen, steht,
- x, y und z für eine ganze Zahl zwischen 0 und 200 stehen, wobei es sich versteht, dass x + y + z von 0 verschieden ist, vorzugsweise wobei x + y + z inklusive zwischen 1 und 150, insbesondere zwischen 20 und 60, liegt;

ii)

$$H-(ALK-O)_x-(*) \text{ und } H-(O-ALK)_x-(*),$$

vorzugsweise $H-(O-ALK)_x-(*)$ ist,
wobei:

- ALK, das gleich oder verschieden sein kann, für eine C1-C6- und insbesondere C1-C4-Ethylengruppe, vorzugsweise Ethylen, steht,
- x für eine ganze Zahl steht, die von 0 verschieden ist, und vorzugsweise zwischen 1 und 200 liegt.

4. Zusammensetzung nach einem der Ansprüche 1 bis 3, wobei das nichtionische Tensid bzw. die nichtionischen Tenside mit einem HLB-Wert bei 25 °C von weniger als 8 und/oder vorzugsweise das nichtionische Tensid bzw. die nichtionischen Tenside mit einem HLB-Wert bei 25 °C größer oder gleich 8 der nachstehenden Formel (I') entspricht bzw. entsprechen:

$$ALK-(O-CH_2-CH_2)_n-OH \text{ (I')}$$

wobei in der Formel (I'):

- ALK für eine $C_8$-$C_{24}$-, vorzugsweise $C_{12}$-$C_{22}$- und weiter bevorzugt $C_{16}$-$C_{18}$-Alkylgruppe steht,

wobei n für eine ganze Zahl steht, die von 0 verschieden ist und für das nichtionische Tensid bzw. die nichtionischen Tenside mit einem HLB-Wert bei 25 °C von weniger als 8 zwischen 1 und 200, vorzugsweise zwischen 1 und 10 und noch besser zwischen 2 und 6 liegt und für das nichtionische Tensid bzw. die nichtionischen Tenside mit einem HLB-Wert bei 25 °C größer oder gleich 8 vorzugsweise streng über 10, insbesondere zwischen 15 und 200, liegt.

5. Zusammensetzung nach einem der Ansprüche 1 bis 4, wobei das nichtionische Tensid bzw. die nichtionischen Tenside mit einem HLB-Wert bei 25 °C von weniger als 8 aus:

- Estern und Ethern von Osen, die gegebenenfalls (poly)oxyalkyleniert, vorzugsweise (poly)oxyalkyleniert, sind;
- Estern von Fettsäuren, insbesondere von $C_8$-$C_{24}$- und vorzugsweise $C_{16}$-$C_{22}$-Fettsäuren, und Polyol, das gegebenenfalls (poly)oxyalkyleniert, vorzugsweise (poly)oxyalkyleniert, ist, insbesondere von (poly)oxyalkyleniertem Glycerin oder von oxyalkyleniertem Sorbitol, vorzugsweise von (poly)-oxyalkyleniertem Glycerin;
- Alkoholen, die gegebenenfalls (poly)oxyalkyleniert, vorzugsweise (poly)oxyalkyleniert, sind;
- und Mischungen davon; vorzugsweise aus Alkoholen, die gegebenenfalls (poly)oxyalkyleniert, vorzugsweise (poly)oxyalkyleniert, sind und vorzugsweise 1 bis 10 Oxyethylen-Einheiten umfassen;

ausgewählt ist bzw. sind.

6. Zusammensetzung nach einem der Ansprüche 1 bis 5, wobei das nichtionische Tensid bzw. die nichtionischen Tenside mit einem HLB-Wert bei 25 °C von weniger als 8 einen Alkohol, der gegebenenfalls (poly)oxyalkyleniert, vorzugsweise (poly)oxyalkyleniert, ist, und einen Ether eines $C_8$-$C_{24}$-Fettalkohols und eines Polyethylenglykols umfasst, wobei der Ether zwischen 1 und 10 und noch besser zwischen 2 und 6 Ethylenglykol-Einheiten umfasst, umfasst bzw. umfassen.

7. Zusammensetzung nach einem der Ansprüche 1 bis 6, wobei das nichtionische Tensid bzw. die nichtionischen Tenside mit einem HLB-Wert bei 25 °C größer oder gleich 8, vorzugwsweise größer oder gelcih 10, aus:

- (poly)oxyalkylenierten Ethern von Glycerin,
- (poly)oxyalkylenierten Alkoholen,
- (poly)oxyalkylenierten Estern von Fettsäuren und Polyethylenglykol,
- (poly)oxyalkylenierten Estern von Fettsäuren und Glycerinethern,
- (poly)oxyalkylenierten Estern von Fettsäuren und Sorbitolethern,

und Mischungen davon; vorzugsweise aus (poly)-oxyalkylenierten Alkoholen, die vorzugsweise mehr als 10 Oxyethylen-Einheiten und insbesondere zwischen 15 und 200 Oxyethylen-Einheiten umfassen, ausgewählt ist bzw. sind.

8. Zusammensetzung nach einem der Ansprüche 1 bis 7, wobei das nichtionische Tensid bzw. die nichtionischen Tenside mit einem HLB-Wert bei 25 °C größer oder gleich 8 einen (poly)oxyalkylenierten Alkohol umfasst bzw. umfassen, der mindestens einen Ether von $C_8$-$C_{24}$-Fettalkohol und Polyethylenglykol umfasst, wobei der Ether mehr als 10 Ethylenglykol-Einheiten und noch besser zwischen 15 und 200 Ethylenglykol-Einheiten umfasst.

9. Zusammensetzung nach einem der vorhergehenden Ansprüche, wobei die wässrige Phase 30 bis 70 Gew.-% und vorzugsweise 40 bis 60 Gew.-%, bezogen auf das Gesamtgewicht der Zusammensetzung, ausmacht.

**10.** Zusammensetzung nach einem der Ansprüche 2 bis 9, wobei das nichtionische Tensid bzw. die nichtionischen Tenside mit einem HLB-Wert bei 25 °C von weniger als 8 der Formel (I) und das nichtionische Tensid bzw. die nichtionischen Tenside mit einem HLB-Wert bei 25 °C größer oder gleich 8 der Formel (I) in einem Gesamtgehalt größer oder gleich 15 Gew.-%, insbesondere zwischen 16 und 40 Gew.-%, noch besser zwischen 18 und 30 Gew.-%, bezogen auf das Gesamtgewicht der Zusammensetzung, vorliegen.

**11.** Zusammensetzung nach einem der vorhergehenden Ansprüche, wobei das nichtionische Tensid bzw. die nichtionischen Tenside mit einem HLB-Wert bei 25 °C von weniger als 8, vorzugsweise der Formel (I), und das nichtionische Tensid bzw. die nichtionischen Tenside mit einem HLB-Wert bei 25 °C größer oder gleich 8, vorzugsweise der Formel (I), in einem solchen jeweiligen Gesamtgehalt vorliegen, dass das Gewichtsverhältnis des nichtionischen Tensids bzw. der nichtionischen Tenside mit einem HLB-Wert bei 25 °C von weniger als 8, vorzugsweise der Formel (I), zu dem nichtionischen Tensid bzw. den nichtionischen Tensiden mit einem HLB-Wert bei 25 °C größer oder gleich 8, vorzugsweise der Formel (I), im Bereich von 1/5 bis 5, vorzugsweise von 1/3 bis 3, vorzugsweise von 2/3 bis 3/2, liegt.

**12.** Zusammensetzung nach einem der vorhergehenden Ansprüche, wobei die Teilchen von filmbildendem Polymer bzw. filmbildenden Polymeren, das bzw. die in Form einer wässrigen Dispersion vorliegt bzw. vorliegen, aus synthetischen Polymeren vom radikalischen Typ oder Polykondensat-Typ, Polymeren natürlicher Herkunft und Mischungen davon ausgewählt sind.

**13.** Zusammensetzung nach einem der vorhergehenden Ansprüche, wobei die Teilchen von filmbildendem Polymer bzw. filmbildenden Polymeren, das bzw. die in Form einer wässrigen Dispersion vorliegt bzw. vorliegen, aus Acrylpolymer-Dispersionen, Polyurethan-Dispersionen, Sulfopolyester-Dispersionen, Vinyl-Dispersionen, Polyvinylacetat-Dispersionen, Vinylpyrrolidon-Dimethylaminopropylmethacrylamid-Lauryldimethylpropylmethacrylamidoammoniumchlorid-Terpolymer-Dispersionen, Dispersionen von Polyurethan/Polyacryl-Hybridpolymeren, Dispersionen von Teilchen vom Kern-Schale-Typ und Mischungen davon, vorzugsweise aus Acrylpolymer-Dispersionen, Dispersionen von Polyurethan/Polyacryl-Hybridpolymeren und Derivaten davon und einer Mischung bzw. Mischungen davon, bevorzugt aus Acrylpolymer-Dispersionen, insbesondere Styrol-Acryl-PolymerDispersionen, und Polyurethan-Dispersionen, insbesondere Polyester-Polyurethan-Dispersionen, und Derivaten davon und einer Mischung bzw. Mischungen davon ausgewählt sind.

**14.** Zusammensetzung nach einem der vorhergehenden Ansprüche, wobei der Gesamtgehalt an Teilchen von filmbildendem Polymer bzw. filmbildenden Polymeren und der Gesamtgehalt des Tensidsystems so beschaffen sind, dass das Gewichtsverhältnis von Teilchen von filmbildendem Polymer bzw. filmbildenden Polymeren zu Tensidsystem kleiner oder gleich 1,75, vorzugsweise kleiner oder gleich 1,5 ist und insbesondere zwischen 1/2 und 1,25 liegt.

**15.** Zusammensetzung nach einem der vorhergehenden Ansprüche, umfassend mindestens ein Farbmittel, das aus einer oder mehreren pulverförmigen Substanzen, vorzugsweise Metalloxiden und insbesondere Eisenoxiden, ausgewählt ist.

**16.** Zusammensetzung nach einem der vorhergehenden Ansprüche mit einer Viskosität bei 25 °C im Bereich von 5 bis 50 Pa.s.

**17.** Verfahren zur Beschichtung von Keratinfasern, insbesondere zum Schminken der Wimpern, umfassend einen Schritt des Aufbringens einer kosmetischen Zusammensetzung zur Beschichtung von Keratinfasern nach einem der vorhergehenden Ansprüche.

**Claims**

**1.** Cosmetic composition for coating keratin fibres, comprising:

- an aqueous phase,
- a surfactant system present in a total content of greater than or equal to 15% by weight relative to the total weight of the composition, said surfactant system comprising:

  i) at least one non-ionic surfactant with an HLB value at 25°C of less than 8, and

ii) at least one non-ionic surfactant with an HLB value at 25°C of greater than or equal to 8, together forming a lamellar phase Lβ,

the non-ionic surfactant (s) with an HLB value at 25°C greater than or equal to 8 is (are) present in a content of greater than or equal to 5% by weight relative to the total weight of the composition, preferably of between 7% and 30%, preferably of between 10% and 20% by weight relative to the total weight of the composition,
the non-ionic surfactant(s) with an HLB value at 25°C of less than 8 is (are) present in a content of greater than or equal to 5% by weight relative to the total weight of the composition, preferably of between 7% and 30%, preferably of between 10% and 20% by weight relative to the total weight of the composition,
the non-ionic surfactant(s) with an HLB value at 25°C of less than 8 and the non-ionic surfactant(s) with an HLB value at 25°C of greater than or equal to 8 are present in a total content of less than or equal to 15%, in particular of between of 16% and 40%, preferably between 18% and 30% by weight, relative to the total weight of the composition,
- at least one aqueous dispersion of particles of at least one film-forming polymer, the film-firming polymer(s) preferably being present in a solids content of greater than or equal to 5% by weight, preferably greater than or equal to 10% by weight, relative to the total weight of said composition,
- it being understood that said composition may comprise at least one wax, the wax(es), when present, being however present in a total content of strictly less than 10% by weight relative to the total weight of the composition.

2. Composition according to Claim 1, in which at least one among the non-ionic surfactant(s) with an HLB value at 25°C of less than 8, and/or, preferably and, at least one among the surfactant(s) with an HLB value at 25°C of greater than or equal to 8 correspond(s) to formula (I) below:

$$(ALK-[C(O)]_a-[O]_b)_c-X \qquad (I)$$

in which formula (I):

- ALK is a $C_7$-$C_{23}$, preferably $C_{11}$-$C_{21}$, more preferentially $C_{15}$-$C_{17}$, alkyl group,
- a and b are integers of between 0 and 100, c is an integer of between 1 and 100, in particular of between 1 and 3, preferably equal to 1, a and b preferably being equal to 0,
- X is a (poly)oxyalkylene group which is optionally substituted and/or terminated with a hydroxyl group, X preferably being an oxyethylene group $(CH_2CH_2O)_n$ or $(OCH_2CH_2)_n$ in which n is greater than or equal to 1, for example between 1 and 200, said (poly)oxyalkylene group preferably being a polyethylene glycol or being the result of at least one substitution of a hydroxyl group, preferably chosen from (poly)glycerols.

3. Composition according to Claim 2, in which the group X is chosen from:

i)

$$HO-(ALK-O)_z-CH2-CH[(OALK)_y-OH]-CH2(O-ALK)_x-(*)$$

in which:

- ALK, which may be identical or different, represent a $C_1$-$C_6$, in particular $C_1$-$C_4$ alkylene group, preferably ethylene,
- x, y and z are an integer of between 0 and 200, it being understood that x+y+z is other than 0, preferably x+y+z being inclusively between 1 and 150, in particular between 20 and 60;

ii)

$$H-(ALK-O)_x-(*) \text{ and } H-(O-ALK)_x-(*),$$

preferably being $H-(O-ALK)_x-(*)$
in which:

- ALK, which may be identical or different, represent a $C_1$-$C_6$, in particular $C_1$-$C_4$, ethylene group, preferably ethylene,
- x is an integer other than 0 and preferably of between 1 and 200.

4. Composition according to any one of Claims 1 to 3, in which the non-ionin surfactant(s) with an HLB value at 25°C of less than 8, and/or, preferably and, the non-ionic surfactant(s) with an HLB value at 25°C of greater than or equal to 8, correspond(s) to formula (I') below:

$$ALK-(O-CH_2-CH_2)_n-OH \qquad (I')$$

in which formula (I'):

- ALK is a $C_8$-$C_{24}$, preferably $C_{12}$-$C_{22}$, more preferentially $C_{16}$-$C_{18}$, alkyl group,

n is an integer other than 0, of between 1 and 200, preferably between 1 and 10, better still between 2 and 6 for the non-ionic surfactant(s) with an HLB value at 25°C of less than 8, and preferably strictly greater than 10, in particular of between 15 and 200, for the non-ionic surfactant(s) with an HLB value at 25°C of greater than or equal to 8.

5. Composition according to any one of Claims 1 to 4, in which the non-ionic surfactant(s) with an HLB value at 25°C of less than 8 is (are) chosen from:

- esters and ethers of optionally (poly)oxyalkylenated, preferably (poly)oxyalkylenated, monosaccharides;
- esters of fatty acids, which are in particular $C_8$-$C_{24}$, and preferably $C_{16}$-$C_{22}$, and of optionally (poly)oxyalkylenated, preferably (poly)oxyalkylenated, polyol, in particular of (poly)oxyalkylenated glycerol or of oxyalkylenated sorbitol, preferably of (poly)oxyalkylenated glycerol;
- optionally (poly)oxyalkylenated, preferably (poly)oxyalkylenated, alcohols;

and mixtures thereof; preferably from optionally (poly)oxyalkylenated, and preferably (poly)oxyalkylenated, alcohols preferably comprising from 1 to 10 oxyethylene units.

6. Composition according to any one of Claims 1 to 5, in which the non-ionic surfactant(s) with an HLB value at 25°C of less than 8 comprise(s) an optionally (poly)oxyalkylenated, preferably (poly)oxyalkylenated, alcohol comprising an ether of a $C_8$-$C_{24}$ fatty alcohol and of polyethylene glycol, said ether comprising between 1 and 10, better still between 2 and 6, ethylene glycol units.

7. Composition according to any one of Claims 1 to 6, in which the non-ionic surfactant(s) with an HLB value at 25°C of greater than or equal to 8, preferably greater than or equal to 10, is (are) chosen from:

- (poly)oxyalkylenated glycerol ethers,
- (poly)oxyalkylenated alcohols,
- esters of a fatty acid and of polyethylene glycol which are (poly)oxyalkylenated,
- esters of a fatty acid and of glycerol ethers which are (poly)oxyalkylenated,
- esters of a fatty acid and of sorbitol ethers which are (poly)oxyalkylenated,

and (a) mixture(s) thereof; preferably from (poly)oxyalkylenated alcohols preferably comprising more than 10 oxyethylene units, in particular between 15 and 200 oxyethylene units.

8. Composition according to any one of Claims 1 to 7, in which the non-ionic surfactant(s) with an HLB value at 25°C of greater than or equal to 8 comprise (s) a (poly)oxyalkylenated alcohol comprising at least one ether of a $C_8$-$C_{24}$ fatty alcohol and of polyethylene glycol, said ether comprising more than 10 ethylene glycol units, better still between 15 and 200 ethylene glycol units.

9. Composition according to any one of the preceding claims, in which the aqueous phase represents from 30% to 70% by weight, preferably from 40% to 60% by weight, relative to the total weight of the composition.

10. Composition according to any one of Claims 2 to 9, in which the non-ionic surfactant(s) with an HLB value at 25°C of less than 8 of formula (I) and the non-ionic surfactant(s) with an HLB value at 25°C of greater than or equal to 8 of formula (I) are present in a total content of greater than or equal to 15%, in particular of between of 16% and 40%, preferably between 18% and 30% by weight, relative to the total weight of the composition.

11. Composition according to any one of the preceding claims, in which the non-ionic surfactant(s) with an HLB value at 25°C of less than 8, preferably of formula (I), and the non-ionic surfactant(s) with an HLB value at 25°C of greater than or equal to 8, preferably of formula (I), are present in a respective total content such that the weight ratio of the non-ionic surfactant(s) with an HLB value at 25°C of less than 8, preferably of formula (I), to the non-ionic surfactant(s) with an HLB value at 25°C of greater than or equal to 8, preferably of formula (I), ranges from 1/5 to 5, preferably from 1/3 to 3, preferably from 2/3 to 3/2.

12. Composition according to any one of the preceding claims, in which the particles of film-forming polymer(s) present in the form of aqueous dispersion(s) are chosen from synthetic polymers, of radical type or of polycondensate type, polymers of natural origin, and mixtures thereof.

13. Composition according to any one of the preceding claims, in which the particles of film-forming polymer(s) present in the form of aqueous dispersion(s) are chosen from acrylic polymer dispersions, polyurethane dispersions, sulfopolyester dispersions, vinyl dispersions, polyvinyl acetate dispersions, dispersions of terpolymer of vinylpyrrolidone, dimethylaminopropylmethacrylamide and lauryldimethyl-propylmethacrylamidoammonium chloride, polyurethane/polyacrylic hybrid polymer dispersions, dispersions of particles of core-shell type, and mixtures thereof, preferably from acrylic polymer dispersions, polyurethane/polyacrylic hybrid polymer dispersions, and derivatives thereof, and mixture(s) thereof, preferentially from dispersions of acrylic polymers, in particular styrene-acrylic polymers, and dispersions of polyurethane, in particular polyester-polyurethane, and derivatives thereof, and mixture(s) thereof.

14. Composition according to any one of the preceding claims, in which the total content of particles of film-forming polymer(s) and the total content of surfactant system are such that the particles of film-forming polymer(s)/surfactant system weight ratio is less than or equal to 1.75, preferably less than or equal to 1.5, in particular between 1/2 and 1.25.

15. Composition according to any one of the preceding claims, comprising at least one colorant chosen from one or more pulverulent material(s), preferably metal oxides, and in particular iron oxides.

16. Composition according to any one of the preceding claims, comprising a viscosity at 25°C ranging from 5 to 50 Pa.s.

17. Method for coating keratin fibres, in particular for making up the eyelashes, comprising a step of applying a cosmetic composition for coating keratin fibres according to any one of the preceding claims.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 1920759 A
- FR 2960151
- US 2003185774 A
- US 5188899 A
- EP 56219 A
- EP 348372 A
- EP 486080 A
- EP 320473 A
- EP 112807 A
- US 3615972 A
- US 5538793 A

- JP 2003128788 B
- JP 2000191789 A
- EP 1579841 A
- US 5002698 A
- US 5874069 A
- US 5919441 A
- US 6051216 A
- US 5981680 A
- WO 2007068371 A
- WO 2008155059 A

**Littérature non-brevet citée dans la description**

- *J. Soc. Cosm. Chem.,* 1954, vol. 5, 249-256
- Encyclopedia of Chemical Technology, KIRK-OTHMER. WILEY, 1979, vol. 22, 333-432

- **C. M. HANSEN.** The three dimensionnal solubility parameters. *J. Paint Technol.,* 1967, vol. 39, 105